# EUROPÄISCHE PATENTSCHRIFT

(11) **EP 3 323 786 B1**
(45) Veröffentlichungstag und Bekanntmachung des Hinweises auf die Patenterteilung: **02.12.2020**
(21) Anmeldenummer: 17169058.9
(22) Anmeldetag: 02.05.2017
(51) Int. Cl.: C01C 1/04, C07C 29/151, C07C 1/10, C01B 3/02, C07C 31/04

(54) **VERFAHREN ZUR KOMBINIERTEN HERSTELLUNG VON METHANOL UND AMMONIAK**
METHOD FOR THE COMBINED PREPARATION OF METHANOL AND AMMONIA
PROCÉDÉ DE FABRICATION COMBINÉE DE MÉTHANOL ET D'AMMONIAC

(30) Priorität: 16.11.2016 EP 16199136
(43) Veröffentlichungstag der Anmeldung: 23.05.2018
(73) Patentinhaber: GasConTec GmbH, 61348 Bad Homburg v. d. Höhe (DE)
(72) Erfinder: Koss Ulrich, 61348 Bad Homburg vor der Höhe (DE); Müller Dierk, 61154 Karben (DE); Wagner Ulrich, 06406 Bernburg (DE)
(74) Vertreter: Patentanwälte Bauer Vorberg Kayser

(56) Entgegenhaltungen:
- EP-A1- 0 011 404
- WO-A1-2016/132092
- US-A1- 2012 148 472

## Beschreibung

Die Erfindung betrifft ein Verfahren zur kombinierten Herstellung von Methanol und Ammoniak mit den Merkmalen des Oberbegriffs von Anspruch 1, ein Verfahren zum Herstellen eines Kraftstoffs gemäß Anspruch 15 sowie eine Anlage zur kombinierten Herstellung von Methanol und Ammoniak mit den Merkmalen des Oberbegriffs von Anspruch 16.

Aus dem Stand der Technik sind verschiedene Ansätze zur kombinierten Herstellung von Methanol und Ammoniak bekannt. Die Offenlegungsschrift EP 0 011 404 beschreibt ein integriertes Verfahren zur Herstellung von Methanol und Ammoniak, bei welchem ein Synthesegas mit Wasserstoff, Kohlenstoffoxid und Stickstoff hergestellt wird, welches dann zunächst einer Methanolsynthese zugeführt wird. Aus dem Restgas werden verbliebene Kohlenstoffoxide entfernt, sodass ein Strom mit Stickstoff und Wasserstoff erhalten wird, welcher Strom einer Ammoniaksynthese zugeführt wird. Nachteilig an diesem Ansatz ist jedoch, dass der in der Methanolsynthese mitgeführte Stickstoff nicht nur groß dimensionierte Kompressoren und Reaktoren erfordert, was die Wirtschaftlichkeit verschlechtert, sondern dabei auch unerwünschte Stoffe wie Trimethylamin entstehen.

Die Offenlegungsschrift DE 10 2004 013 539 A1 beschreibt ebenfalls ein Verfahren zur Koproduktion von Methanol und Ammoniak. Hier wird das Synthesegas durch katalytische partielle Oxidation mit Sauerstoff aus einer Lufttrennungseinheit gewonnen, sodass der erhebliche Stickstoffanteil in der Methanolsynthese entfällt. Dieses Synthesegas wird dann jeweils prozesstechnisch parallel angeordneten jeweiligen Reaktoranordnungen für die Methanolsynthese und die Ammoniaksynthese zugeführt. Nachteilig ist hier, dass der Reaktoranordnung für die Ammoniaksynthese vorgelagert eine aufwändige Apparatur zur Entfernung von Methan sowie von Argon und verbliebenem Kohlenstoffmonoxid erforderlich ist, und zwar speziell eine die Wirtschaftlichkeit beeinträchtigende Tieftemperaturzerlegung.

Die Patentschrift US 5,180,570 beschreibt ebenfalls einen integrierten Prozess zur Herstellung von Methanol und Ammoniak aus einem kohlenstoffhaltigen Energieträgerstrom. Das Synthesegas wird in einer mehrstufigen Reformeranordnung mit einer herkömmlichen Dampfreformierung und einer nachgelagerten autothermen Reformierung unter Zufuhr von Sauerstoff gewonnen. Nachteilig an diesem Stand der Technik ist, dass die Dampfreformierung, welche eine Spaltung des Methans mit Wasser in ein wasserstoffreiches Synthesegas zum Erreichen des erforderlichen Wasserstoffüberschusses bei der Methanolsynthese bewirkt, nur bei vergleichsweise geringen Drücken durchgeführt werden kann. Daher ist eine aufwändige Kompression des gesamten Synthesegases vor der Methanolsynthese erforderlich. Ferner wird in der einstufigen Methanolsynthese keine hinreichend umfassende Umsetzung der Kohlenstoffe zu Methanol erreicht, weswegen sowohl eine Kompression von unreagiertem Recyclegas als auch eine ebenfalls Tieftemperaturreinigung des Restgases vor der Ammoniaksynthese erforderlich ist. Beide Maßnahmen erhöhen den Aufwand wesentlich.

Die internationale Patentanmeldung WO 2016/132092 A1 beschreibt ein Verfahren zur Herstellung von Formaldehyd-stabilisiertem Harnstoff in welchem ein Synthesegas erzeugt wird, Kohlendioxid aus dem Synthesegas gewonnen wird, Methanol aus dem an Kohlendioxid abgereicherten Synthesegas synthetisiert wird, ein Teil des gewonnenen Methanols einer Oxidation mit Luft in einer Formaldehyd-Produktionseinheit unterzogen wird und ein Methanolsyntheseabgas methanisiert wird, um ein Ammoniaksynthesegas zu bilden.

Die europäische Patentanmeldung EP 0 011 404 beschreibt ein Verfahren zur Herstellung von Methanol und Ammoniak. Das Verfahren umfasst das Erzeugen eines stickstoffhaltigen Synthesegases, das unvollständige Reagieren der Kohlenstoffoxide und des Wasserstoffs zu Methanol und das Weiterleiten des unreagierten Gases zu der Ammoniaksynthese. Das Verfahren ist dadurch gekennzeichnet, dass die Methanolsynthese in einer ersten, dampffreien Stufe und einer zweiten Stufe durchgeführt wird, in welcher zweiten Stufe ausreichend Dampf vorhanden ist, um im Wesentlichen das gesamte nicht zu Methanol umgewandelte Kohlenstoffmonoxid in Kohlenstoffdioxid umzuwandeln.

Die amerikanische Offenlegungsschrift US 2012/0148472 A1, welche als nächstkommend angesehen wird, beschreibt ein Verfahren zur kombinierten Herstellung von Methanol und Ammoniak, wobei ein Synthesegas im Wesentlichen bestehend aus Kohlenstoffmonoxid, Kohlenstoffdioxid und Wasserstoff zunächst Ausgehend von diesem Stand der Technik besteht die Aufgabe der Erfindung folglich darin, ein Verfahren zur kombinierten Herstellung von Methanol und von Ammoniak bereitzustellen, welches wirtschaftlicher ist.

Bezogen auf ein Verfahren zur kombinierten Herstellung von Methanol und Ammoniak mit den Merkmalen des Oberbegriffs von Anspruch 1 wird diese Aufgabe durch die Merkmale des kennzeichnenden Teils von Anspruch 1 gelöst. Bezogen auf ein Verfahren zum Herstellen eines Kraftstoffs wird diese Aufgabe durch die Merkmale des kennzeichnenden Teils von Anspruch 15 gelöst. Bezogen auf eine Anlage zur kombinierten Herstellung von Methanol und Ammoniak mit den Merkmalen des Oberbegriffs von Anspruch 16 wird diese Aufgabe durch die Merkmale des kennzeichnenden Teils von Anspruch 16 gelöst.

Der Erfindung liegt die Erkenntnis zugrunde, dass der bei der Synthesegasherstellung unter hohem Druck mit Sauerstoff auftretende zu geringe Anteil von Wasserstoff - sogar für die Methanolsynthese zu gering - dadurch kompensiert werden kann, dass nach der Herstellung des Synthesegases, aber vor der Zuführung des Synthesegases zu der Methanolsynthese, der Wasserstoffanteil im Synthesegas angereichert werden kann. Diese Anreicherung macht die sehr komplexe und aufwändige Dampfreformierungsstufe entbehrlich, welche herkömmlicherweise für die Bereitstellung von einer für die Methanolsynthese und für die Ammoniaksynthese ausreichenden Menge von Wasserstoff im Synthesegasstrom erforderlich ist.

Durch das Entfallen der Dampfreformierungsstufe wiederum kann sowohl die Synthesegasherstellung selbst als auch die obige Anreicherung - sei es nun die Zufuhr von Wasserstoff oder das Entfernen von Kohlenstoffoxiden - bei Druckverhältnissen erfolgen, dank derer eine Kompression vor der Methanolsynthese entfallen oder jedenfalls kleiner dimensioniert werden kann. Ferner ist es durch die obige Anreicherung möglich, bei der Methanolsynthese eine solch umfassende Umsetzung von Kohlenstoffmonoxid bereits in einem Durchlauf zu erreichen, dass auch die Notwendigkeit für einen Recyclekompressor bei der Methanolsynthese entfällt. Auf diese Weise können die regelmäßig für die Wirtschaftlichkeit der Methanolsynthese wichtigsten Faktoren optimiert werden.

Der Unteranspruch 2 beschreibt eine besonders vorteilhafte Möglichkeit zur Anreicherung von Wasserstoff durch Entfernen von Kohlenstoffdioxid. Denn dies erhöht nicht nur den molaren Anteil von Wasserstoff direkt, sondern reduziert gleichermaßen auch den Verbrauch von Wasserstoff in der Methanolsynthese durch Kohlenstoffdioxid.

Demgegenüber betreffen die Unteransprüche 3 und 4 die Möglichkeit, die relative Gewichtung der Methanolherstellung einerseits und der Ammoniakherstellung andererseits zu verschieben.

Die Unteransprüche 6 bis 10 beschreiben eine bevorzugte Ausgestaltung mit einer zweistufigen Methanolsynthese, welche besonders dazu geeignet ist, bereits in einem einzigen Durchlauf - auch als "once through"-Betrieb bezeichnet, eine weit gehende Umsetzung des Kohlenstoffmonoxids in Methanol zu erreichen. Die speziell in dem Unteranspruch 10 beschriebene Anordnung eines Boosterkompressors zwischen den Methanolsynthesestufen kann eine Kompression vor der Ammoniaksynthese entbehrlich machen.

Die Unteransprüche 11 und 12 beziehen sich auf wesentliche Merkmale der autothermen Reformierung als vorteilhaften Ansatz zum Gewinnen des Synthesegasstroms.

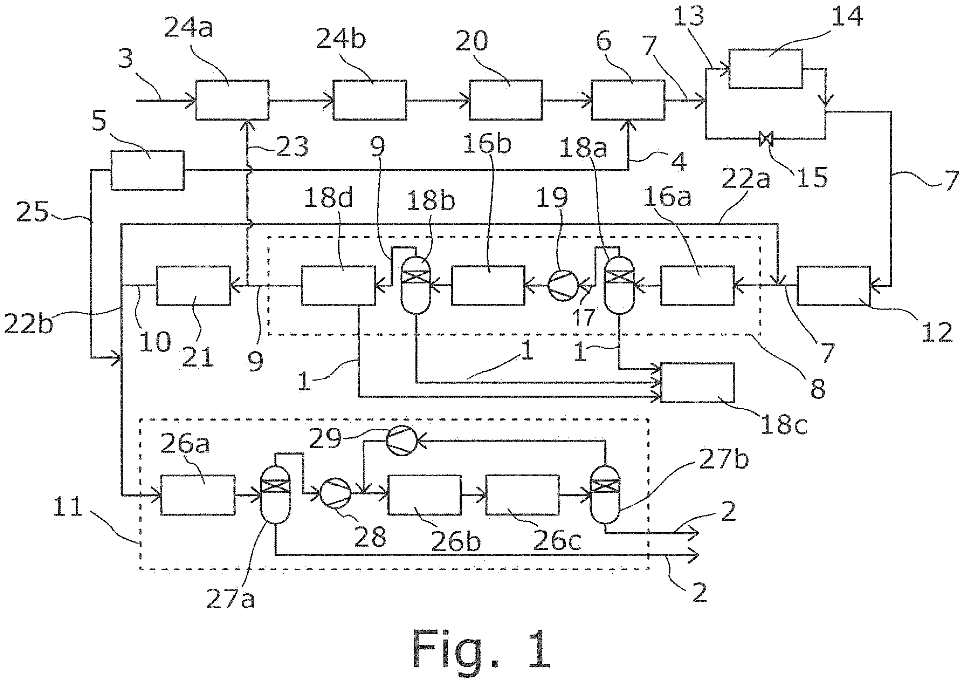

Weitere Einzelheiten, Merkmale, Ziele und Vorteile der vorliegenden Erfindung werden nachfolgend anhand der lediglich ein Ausführungsbeispiel einer vorschlagsgemäßen Anlage wiedergebenden Zeichnung erläutert. Ferner werden drei Ausführungsbeispiele des vorschlagsgemäßen Verfahrens zur kombinierten Herstellung von Methanol und von Ammoniak beschrieben. In der Zeichnung zeigt
- Fig. 1: schematisch das Fließbild eines Ausführungsbeispiels einer vorschlagsgemäßen Anlage zur kombinierten Herstellung von Methanol und Ammoniak.

Die vorschlagsgemäße Anlage, deren Fließbild in der Fig. 1 schematisch dargestellt ist, dient der kombinierten Herstellung von Methanol 1 und von Ammoniak 2 und ist ebenso zur Ausführung des vorschlagsgemäßen Verfahrens zur kombinierten Herstellung von Methanol 1 und von Ammoniak 2 eingerichtet.

Bei dem vorschlagsgemäßen Verfahren zur kombinierten Herstellung von Methanol 1 und von Ammoniak 2 wird ein kohlenstoffhaltiger Energieträgerstrom 3, bei welchem es sich hier speziell um einen Erdgasstrom handelt, sowie ein Sauerstoffstrom 4 aus einer Sauerstoffgewinnungsanordnung 5 einer Synthesegasreaktoranordnung 6 zum Gewinnen eines Synthesegasstroms 7 zugeführt. Bei dieser Sauerstoffgewinnungsanordnung 5 handelt es sich um eine im Prinzip beliebige Anlage oder Vorrichtung, welche insbesondere aus einem Fluid - also aus einer Flüssigkeit oder aus einem Gas, bzw. aus einem Flüssigkeitsgemisch oder aus einem Gasgemisch - molekularen Sauerstoff gewinnt. Dies kann insbesondere dadurch erfolgen, dass ein sauerstoffhaltiges Fluid in seine Bestandteile zerlegt wird. Insbesondere kann diese Sauerstoffgewinnungsanordnung 5 - wie im Ausführungsbeispiel der Fig. 1 - eine Luftzerlegung (air separation unit - ASU) zum Gewinnen von molekularem Sauerstoff aus der Luft sein.

Sowohl diese Sauerstoffgewinnungsanordnung 5 als auch die Synthesegasreaktoranordnung 6 sind von der vorschlagsgemäßen Anlage zur kombinierten Herstellung von Methanol 1 und von Ammoniak 2 umfasst. Da aus der Sauerstoffgewinnungsanordnung 5 gewonnen, weist der Sauerstoffstrom 4 als überwiegenden Bestandteil Sauerstoff auf. In jedem Falle übersteigt der Sauerstoffanteil des Sauerstoffstroms 4 denjenigen der Umgebungsluft und der Stickstoffanteil des Sauerstoffstroms 4 liegt unter demjenigen der Umgebungsluft. Vorschlagsgemäß beträgt der Sauerstoffanteil an dem Sauerstoffstrom 4 mindestens 80 %, weiter vorzugsweise mindestens 90 % und insbesondere mindestens 95 %. Ebenso kann es sein, dass der Sauerstoffstrom 4 im Wesentlichen aus Sauerstoff besteht, wobei geringe Anteile insbesondere von Stickstoff und Edelgasen in dem Sauerstoffstrom 4 vorhanden sein können.

Vorschlagsgemäß umfasst der Synthesegasstrom 7 Wasserstoff und Kohlenstoffoxide, also Kohlenstoffmonoxid sowie Kohlenstoffdioxid. Der Synthesegasstrom 7 kann dabei in an sich beliebiger Weise mit aus dem Stand der Technik bekannten Verfahren zur Synthesegasherstellung unter Zufuhr eines Sauerstoffstroms 4 aus der Sauerstoffgewinnungsanordnung 5 gewonnen werden. Die bevorzugte Art der Synthesegasherstellung wird untenstehend beschrieben. Daneben kann der Synthesegasstrom 7 auch weitere Stoffe wie etwa Methan, Stickstoff und Edelgase in kleinen Anteilen aufweisen, vorzugsweise mit einem jeweiligen molaren Anteil von weniger als 10 %.

Gemäß dem vorschlagsgemäßen Verfahren wird der Synthesegasstrom 7 einer Methanol-Reaktoranordnung 8 zur teilweisen Umwandlung in Methanol 1 zugeführt und es wird aus der Methanol-Reaktoranordnung 8 ein Restgasstrom 9 erhalten. Die teilweise Umwandlung in Methanol 1 kann insbesondere katalytisch erfolgen. Der Restgasstrom 9 kann Methanol sowie unreagierte Bestandteile des Synthesegasstroms 7 umfassen, insbesondere also Wasserstoff und Kohlenstoffoxide. Auch die Methanol-Reaktoranordnung 8 ist von der vorschlagsgemäßen Anlage umfasst. Das vorschlagsgemäße Verfahren sieht dann weiter vor, dass aus dem Restgasstrom 9 ein wasserstoffhaltiger Strom 10 gewonnen wird, welcher wasserstoffhaltige Strom 10 zumindest teilweise - vorzugsweise im Wesentlichen vollständig - einer Ammoniak-Reaktoranordnung 11, welche ebenso von der vorschlagsgemäßen Anlage umfasst ist, zugeführt und dort zumindest teilweise - wiederum vorzugsweise im Wesentlichen vollständig - in Ammoniak 2 umgewandelt wird. Ebenso kann die Umwandlung in Ammoniak 2 katalytisch erfolgen.

Das vorschlagsgemäße Verfahren ist nun dadurch gekennzeichnet, dass in einem Anreicherungsschritt der molare Anteil an Wasserstoff des aus der Synthesegasreaktoranordnung 6 gewonnenen Synthesegasstroms 7 gegenüber dem molaren Anteil an Kohlenstoffoxiden vor Zuführung zu der Methanol-Reaktoranordnung 8 erhöht wird. Mit anderen Worten ist der molare Anteil an Wasserstoff im Synthesegasstrom 7 gegenüber dem molaren Anteil der Kohlenstoffoxide im Synthesegasstrom 7 nach dem Anreicherungsschritt, welcher prozesstechnisch zwischen der Synthesegasreaktoranordnung 6 und der Methanol-Reaktoranordnung 8 erfolgt, höher als vor dem Anreicherungsschritt.

Grundsätzlich kann diese Anreicherung von Wasserstoff im Synthesegasstrom 7 - welche sich auf den molekularen Wasserstoff (H₂) bezieht - auf beliebige Art und Weise erfolgen. So kann einerseits zusätzlicher Wasserstoff dem Synthesegasstrom 7 zugeführt werden. Entsprechend ist es bevorzugt, dass der molare Anteil an Wasserstoff durch - insbesondere nur durch - eine Zufuhr von Wasserstoff zu dem Synthesegasstrom 7 erhöht wird.

Andererseits können auch Kohlenstoffoxide aus dem Synthesegasstrom 7 entfernt werden. Dementsprechend kann es bevorzugt sein, dass der molare Anteil an Wasserstoff durch - insbesondere nur durch - ein zumindest teilweises Entfernen von Kohlenstoffoxiden - also speziell von Kohlenstoffmonoxid und/oder von Kohlenstoffdioxid - aus dem Synthesegasstrom 7 erhöht wird. Darüber hinaus weist die Anlage eine Druckwechsel-Adsorptionsanlage (PSA) 21 auf, welcher der Restgasstrom 9 zum Gewinnen des wasserstoffhaltigen Stroms 10 zugeführt wird. Diese PSA 21, welche von der vorschlagsgemäßen Anlage umfasst ist, kann auch zum Ausschleusen eines Purgegases dienen.

Eine bevorzugte Ausgestaltung sieht vor, dass in dem Anreicherungsschritt Kohlenstoffdioxid zumindest teilweise aus dem Synthesegasstrom 7 entfernt wird. Folglich erhöht sich die molare Menge des Wasserstoffs im Synthesegasstrom 7 nicht, sondern nur sein molarer Anteil. Das schlägt sich nieder in einer deutlichen Erhöhung der Stöchiometriezahl von weit unter 2 bis weit über 3. Hierzu kann vorzugsweise zumindest ein Teilstrom des Synthesegasstroms 7 - vorzugsweise entsprechend dem Ausführungsbeispiel der vorschlagsgemäßen Anlage der Fig. 1 der im Wesentlichen gesamte Synthesegasstrom 7 - einer Kohlenstoffdioxidwäsche 12 - bei welcher es sich vorzugsweise um eine Anordnung zur Kohlenstoffdioxidwäsche der vorschlagsgemäßen Anlage handelt - zur zumindest teilweisen Entfernung des Kohlenstoffdioxids zugeführt werden. Dabei kann die Wäsche selbst auf eine beliebige, aus dem Stand der Technik an sich bekannte Weise erfolgen.

Gemäß einer ersten Variante wird in dem Anreicherungsschritt und insbesondere bei der Kohlenstoffdioxidwäsche 12 das Kohlenstoffdioxid aus dem Synthesegasstrom 7 durch eine chemische Wäsche entfernt. Hierzu zählt bevorzugt eine chemische Wäsche jeweils mit Piperazin-aktiviertem Methyldiethanolamin, mit einfachem Methyldiethanolamin, mit einer wässrigen Ammoniaklösung oder mit einer wässrigen Soda-Lösung. Gemäß einer zweiten Variante wird in dem Anreicherungsschritt das Kohlenstoffdioxid aus dem Synthesegasstrom 7 durch ein physikalisch absorbierendes Absorptionsmittel entfernt. Vorzugsweise handelt es sich bei dem physikalisch absorbierenden Absorptionsmittel um kaltes Methanol, um Selexol oder um N-Methyl-2-pyrrolidon (NMP). Im Falle dass es sich bei dem physikalisch absorbierenden Absorptionsmittel um kaltes Methanol handelt, kann es sich um aus der Methanol-Reaktoranordnung 8 gewonnenes Methanol handeln.

Es ist nicht erforderlich, dass in der obigen Kohlenstoffdioxidwäsche 12 eine besondere Reinheit erreicht wird. Daher kann die Kohlenstoffdioxidwäsche 12 - insbesondere im Falle, dass ein physikalisch absorbierendes Absorptionsmittel zum Einsatz kommt - auch ohne dampfgetriebene Regeneration ausgestaltet werden. Bevorzugt ist daher, dass die Kohlenstoffdioxidwäsche 12 eine druckentspannungsbasierte Regenerationsstufe aufweist. Mit anderen Worten wird ein Waschmittel der Kohlenstoffdioxidwäsche 12 durch Druckentspannung regeneriert.

Bevorzugt ist weiter, dass der der Methanol-Reaktoranordnung 8 zugeführte Synthesegasstrom 7 - also der Synthesegasstrom 7 nach dem Anreicherungsschritt - einen molaren Anteil von Kohlenstoffdioxid von bis zu 3 % aufweist. Folglich wird der molare Anteil von Kohlenstoffdioxid in dem Synthesegasstrom 7 auf einen Wert reduziert, welcher deutlich niedriger als bei einer herkömmlichen Kohlenstoffdioxidwäsche ist.

Prinzipiell kann es sein, dass der Anreicherungsschritt nur aus dem obigen Entfernen von Kohlenstoffdioxid durch die Kohlenstoffdioxidwäsche 12 besteht. Dieses Vorgehen ist für die Methanolsynthese besonders vorteilhaft, da Kohlenstoffdioxid in der Methanolsynthese mehr Wasserstoff verbraucht als Kohlenstoffmonoxid. Wenn sich das Verhältnis der Produktion von Methanol zu Ammoniak mehr zu Ammoniak verschieben soll, kann es aber sein, dass der Anreicherungsschritt alternativ oder zusätzlich andere Maßnahmen zur Anreicherung von Wasserstoff umfasst. Eine hier bevorzugte Variante sieht vor, dass in dem Anreicherungsschritt zumindest ein - insbesondere weiterer - Teilstrom 13 des Synthesegasstroms 7 einer Shiftkonvertierung 14 zugeführt wird, in welcher Kohlenstoffmonoxid zumindest teilweise durch Reaktion mit Wasserdampf zu Wasserstoff und Kohlenstoffdioxid umgewandelt wird. Da als Ergebnis dieser Reaktion die Zahl der Kohlenstoffoxidmoleküle gleichbleibt - wiewohl aus einem Molekül Kohlenstoffmonoxid ein Molekül Kohlenstoffdioxid wird - und sich gleichzeitig die Zahl der Wasserstoffmoleküle erhöht, erhöht sich im Ergebnis auch der molare Anteil an Wasserstoff. Es kann auch der Synthesegasstrom 7 im Wesentlichen insgesamt der Shiftkonvertierung 14 zugeführt werden. Der in der Shiftkonvertierung 14 auftretende Vorgang wird auch als Wassergas-Shift-Reaktion bezeichnet. Folglich kann die Shiftkonvertierung 14, welche vorzugsweise von der vorschlagsgemäßen Anlage umfasst ist, auch als Vorrichtung für eine Wassergas-Shift-Reaktion bezeichnet werden. Auch diese Wassergas-Shift-Reaktion führt zu einer Erhöhung des molaren Anteils von Wasserstoff im Synthesegasstrom 7 gegenüber dem molaren Anteil der Kohlenstoffoxide.

Bevorzugt ist weiter, dass - gemäß der Darstellung in der Fig. 1 - die Abzweigung des Teilstroms 13 zur Shiftkonvertierung 14 der Kohlenstoffdioxidwäsche 12 prozesstechnisch vorgelagert ist. Entsprechend ist es bevorzugt, dass der der Shiftkonvertierung 14 zugeführte Teilstrom 13 anschließend der Kohlenstoffdioxidwäsche 12 zugeführt wird. Auf diese Weise kann also das bei der Wassergas-Shift-Reaktion angefallene Kohlenstoffdioxid in der Kohlenstoffdioxidwäsche 12 wieder ausgewaschen werden. Insofern findet also eine zweifache Erhöhung des molaren Anteils des Wasserstoffs statt. Weiter ist bei der Shiftkonvertierung 14 bevorzugt, dass die Shiftkonvertierung 14 im Wesentlichen ausschließlich eine Hochtemperatur-Wassergas-Shift-Reaktion, und zwar vorzugsweise bei mindestens 450°, durchführt. Auf diese Weise kann eine separate Stufe für eine Niedertemperatur-Wassergas-Shift-Reaktion entfallen.

Die durch die Wassergas-Shift-Reaktion und durch die nachfolgende Kohlenstoffdioxidwäsche 12 bewirkte Änderung der jeweiligen molaren Anteile des Wasserstoffs, des Kohlenstoffmonoxids und des Kohlenstoffdioxids in dem Synthesegasstrom 7 beeinflusst sowohl die Umwandlung zu Methanol 1 in der Methanol-Reaktoranordnung 8 als auch die Umwandlung zu Ammoniak 2 in der Ammoniak-Reaktoranordnung 11. Folglich kann in einer bevorzugten Ausführungsform der molare Anteil des der Shiftkonvertierung 14 zugeführten insbesondere weiteren Teilstroms 13 am Synthesegasstrom 7 zur Einstellung des Verhältnisses des durch Umwandlung gewonnenen Methanols 1 und des durch Umwandlung gewonnen Ammoniaks 2 angepasst werden. Die vorschlagsgemäße Anlage weist hierzu vorzugsweise das in der Fig. 1 gezeigte Steuerventil 15 zur Einstellung dieses Verhältnisses auf.

In einem ersten Ausführungsbeispiel des vorschlagsgemäßen Verfahrens zur kombinierten Herstellung von Methanol 1 und von Ammoniak 2 - hier ausgeführt durch das gezeigte Ausführungsbeispiel der vorschlagsgemäßen Anlage - und bei allen diesem ersten Ausführungsbeispiel des vorschlagsgemäßen Verfahrens zugeordneten untenstehenden Mengenangaben wird jedoch kein Teilstrom 13 zur Shiftkonvertierung 14 abgezweigt. Vielmehr ist das Steuerventil 15 so eingestellt, dass möglichst viel Methanol gegenüber Ammoniak hergestellt wird und somit der im Wesentlichen vollständige Synthesegasstrom 7 an der Shiftkonvertierung 14 herumgeführt wird. Soll das obige Verhältnis zugunsten von Ammoniak verschoben werden, so kann ein entsprechend größerer Anteil des Synthesegasstroms 7 als Teilstrom 13 der Shiftkonvertierung 14 zugeführt werden.

In diesem ersten Ausführungsbeispiel des vorschlagsgemäßen Verfahrens zur kombinierten Herstellung von Methanol 1 und Ammoniak 2 weist der der Methanol-Reaktoranordnung 8 zugeführte Synthesegasstrom 7 - also nach fakultativer, hier nicht erfolgter Shiftkonvertierung 14 und Kohlenstoffdioxidwäsche 12 - bei einem Druck von im Wesentlichen 77 bar und einer Temperatur von 40 °C im Wesentlichen einen jeweiligen molaren Anteil von 68 % Wasserstoff, 24 % Kohlenstoffmonoxid, 2 % Kohlenstoffdioxid, 2 % Methan, 2 % Wasser und 1 % Stickstoff auf. Dabei ist der Beitrag eines ggf. rückgeführten ersten Wasserstoff-Teilstroms 22a - welcher untenstehend noch beschrieben wird - nicht berücksichtigt. Es handelt sich also um den der Kohlenstoffdioxidwäsche 12 prozesstechnisch unmittelbar nachgelagerten Synthesegasstrom 7.

Diese z. B. durch die oben beschriebenen Maßnahmen erreichte Anreicherung des molaren Anteils an Wasserstoff des Synthesegasstroms 7 erlaubt es, eine weit gehende Umwandlung des Kohlenstoffmonoxids in der Methanol-Reaktoranordnung 8 zu erreichen. So ist es bevorzugt, dass Kohlenstoffmonoxid in dem der Methanol-Reaktoranordnung 8 zugeführten Synthesegasstrom 7 im Wesentlichen vollständig in Methanol 1 umgewandelt wird, sodass der molare Anteil von Kohlenstoffmonoxid im Restgasstrom 9 höchstens 4 % beträgt. Im ersten Ausführungsbeispiel des vorschlagsgemäßen Verfahrens zur kombinierten Herstellung von Methanol 1 und Ammoniak 2 weist der Restgasstrom 9 - und zwar speziell vor Zuführung zu der unten noch zu beschreibenden Druckwechsel-Adsorptionsanlage 21 - bei einem Druck von im Wesentlichen 87 bar und bei einer Temperatur von 43 °C im Wesentlichen einen jeweiligen molaren Anteil von 90 % Wasserstoff, 4 % Kohlenstoffmonoxid, 1 % Kohlenstoffdioxid, 2 % Methan, 1 % Wasser und 1 % Stickstoff auf.

Grundsätzlich kann die Methanol-Reaktoranordnung 8 beliebig ausgestaltet sein. Bevorzugt ist es, dass - gemäß der Darstellung in der Fig. 1 - die Methanol-Reaktoranordnung 8 eine erste Reaktorstufe 16a zur insbesondere katalytischen Synthese von Methanol 1 und eine zweite Reaktorstufe 16b zur insbesondere katalytischen Synthese von Methanol 1 aufweist, dass der der Methanol-Reaktoranordnung 8 zugeführte Synthesegasstrom 7 der ersten Reaktorstufe 16a zugeführt wird und dass ein unreagiertes Restgas 17 aus der ersten Reaktorstufe 16a der zweiten Reaktorstufe 16b zugeführt wird. Für den oben erwähnten hohen Grad an Umwandlung zu Methanol 1 ist es bevorzugt, dass das durch S gemäß der Formel S = (n(H₂) - n(CO₂) ) / (n(CO) + n(CO₂)) mit n in [mol] gegebene molare Verhältnis in der ersten Reaktorstufe 16a von mindestens 2,5, vorzugsweise von mindestens 3,5 erreicht wird. Dieses molare Verhältnis wird auch als Stöchiometriezahl bezeichnet. Die Stöchiometriezahl kann durch besonders weit gehende Entfernung des Kohlenstoffdioxids in der Kohlenstoffdioxidwäsche 12 - ggf. in Kombination mit einer vorgeschalteten Shiftkonvertierung 14 - erhöht werden.

Prinzipiell kann sowohl die erste Reaktorstufe 16a als auch die zweite Reaktorstufe 16b beliebig ausgeführt sein. Bevorzugt ist jedoch, dass eine oder beide der Reaktorstufen 16a, b gekühlt werden. Vorzugsweise wird also der ersten Reaktorstufe 16a ein erstes Fluid - z. B. Wasser - zu seiner Kühlung zugeführt und alternativ oder zusätzlich der zweiten Reaktorstufe 16b ein zweites Fluid, bei dem es sich ebenfalls um Wasser handeln kann, zu seiner Kühlung zugeführt. So kann es sein, dass die erste Reaktorstufe 16a isotherm betrieben wird und alternativ oder zusätzlich die zweite Reaktorstufe 16b isotherm betrieben wird. Speziell der isotherme Betrieb beider Reaktorstufen 16a, b - was dem in der Fig. 1 gezeigten Ausführungsbeispiel entspricht - erlaubt eine mengenmäßig in etwa gleiche Synthese von Methanol 1 in beiden Reaktorstufen 16a, b. So ist es bevorzugt, dass zwischen 30 % und 70 %, insbesondere zwischen 40 % und 60 %, der molaren Menge an in der Methanol-Reaktoranordnung 8 durch Umwandlung gewonnenem Methanol 1 in der ersten Reaktorstufe 16a und die restliche molare Menge an in der Methanol-Reaktoranordnung 8 durch Umwandlung gewonnenem Methanol 1 in der zweiten Reaktorstufe 16b anfällt.

Im ersten Ausführungsbeispiel des vorschlagsgemäßen Verfahrens zur kombinierten Herstellung von Methanol 1 und Ammoniak 2 wird aus einer - unten noch zu beschreibenden - ersten Kondensationsstufe 18a ein Massenstrom von 94.962 kg/h Methanol 1 gewonnen und aus einer - ebenfalls jeweils unten noch zu beschreibenden - zweiten Kondensationsstufe 18b und einer Wäschervorrichtung 18d - ein Massenstrom von insgesamt ca. 72.043 kg/h Methanol 1 gewonnen. Insgesamt ergibt das einen Massenstrom an gewonnenem Methanol 1 von Methanol 1 aus den Kondensationsstufen 18a, b und der Wäschervorrichtung 18d von 167.005 kg/h. Dieses Methanol 1 wird dabei zunächst als Teil eines jeweiligen Rohmethanolstroms gewonnen, welcher Rohmethanolstrom auch einen bestimmten Anteil an Wasser aufweisen kann. Auch wenn diese beiden obigen Angaben nicht notwendigerweise eins-zu-eins den Reaktorstufen 16a, b zugeordnet werden können, so ist doch eine im Wesentlichen vorliegende Balance erkennbar. Weitere relevante Mengenangaben zu diesem ersten Ausführungsbeispiel folgen untenstehend. Herkömmliche zweistufige Reaktoranordnungen zur Methanolsynthese sind hingegen regelmäßig dadurch gekennzeichnet, dass in der jeweils nachgelagerten Reaktorstufe deutlich weniger Methanol umgewandelt wird.

Durch die große Menge des gewonnenen Methanols 1 in beiden Reaktorstufen 16a, b wird es möglich, speziell die Kohlenstoffoxide des Synthesegasstroms 7 nur einmal beide Reaktorstufen 16a, b durchlaufen zu lassen.

Entsprechend ist es bevorzugt, dass das der zweiten Reaktorstufe 16b zugeführte unreagierte Restgas 17 im Wesentlichen alle unreagierten Kohlenstoffoxide aus der ersten Reaktorstufe 16a umfasst. Es gibt also im Wesentlichen keine unreagierten Kohlenstoffoxide aus dem unreagierten Restgas 17, welche wieder in die erste Reaktorstufe 16a zurückgeführt werden. Mit anderen Worten ist es bevorzugt, dass Kohlenstoffoxide des Synthesegasstroms 7 die erste Reaktorstufe 16a nur einmal durchlaufen. Ein ins Gewicht fallender Recyclekompressor für die Methanolsynthese kann dadurch entfallen. Dies bedeutet aber nicht nur die Ersparnis des Recyclekompressors an sich, sondern ermöglicht auch eine Verkleinerung der Methanol-Reaktoranordnung 8 bzw. ihrer Reaktorstufen 16a, b, da durch die fehlende Zirkulation durch den Recyclekompressor die Materialvolumina insgesamt erheblich reduziert werden.

Vorteilhafterweise gilt ein solcher, nur einmaliger Durchlauf auch für die zweite Reaktorstufe 16b. Die entsprechende bevorzugte Ausführungsform, welche auch der Darstellung der Fig. 1 entspricht, ist dadurch gekennzeichnet, dass der Restgasstrom 9 im Wesentlichen alle unreagierten Kohlenstoffoxide aus der zweiten Reaktorstufe 16b umfasst. Aus dem Restgasstrom 9 wird ja der wasserstoffhaltige Strom 10 gewonnen. Mit anderen Worten gibt es im Wesentlichen keine unreagierten Kohlenstoffoxide aus der zweiten Reaktorstufe 16b, welche zu einer der beiden Reaktorstufen 16a, b rückgeführt werden. Vorzugsweise durchlaufen Kohlenstoffoxide die zweite Reaktorstufe 16b nur einmal.

Ein Entfernen von Methanol 1 zwischen den beiden Reaktorstufen 16a, b ist ebenso äußerst vorteilhaft zur Verbesserung der Methanolumwandlung in der zweiten Reaktorstufe 16b. Daher ist es bevorzugt, dass der ersten Reaktorstufe 16a prozesstechnisch nachgelagert eine erste Kondensationsstufe 18a zur Abtrennung von in der ersten Reaktorstufe 16a umgewandeltem Methanol 1 und zum Erhalten des unreagierten Restgases 17 aus der ersten Reaktorstufe 16a ist. Diese erste Kondensationsstufe 18a ist von der Methanol-Reaktoranordnung 8 umfasst. Das Methanol 1 kann dabei - wie oben erwähnt - als ein Rohmethanol abgetrennt werden, welches dann einer Destillationsvorrichtung 18c zum Gewinnen von Reinmethanol zugeführt werden kann, welche Destillationsvorrichtung 18c ebenfalls von der vorschlagsgemäßen Anlage umfasst ist. Im ersten Ausführungsbeispiel des vorschlagsgemäßen Verfahrens zur kombinierten Herstellung von Methanol 1 und Ammoniak 2 wird aus der Destillationsvorrichtung 18c ein im Wesentlichen aus Methanol bestehender Reinmethanolstrom mit einem Massenstrom von 166.785 kg/h gewonnen, wobei sich die geringe Differenz dieses Massenstroms an Methanol zu der Summe der oben genannten Massenströme durch Verluste erklärt.

Zur Entfernung des Methanols 1 auch aus der zweiten Reaktorstufe 16b ist bevorzugt vorgesehen, dass der zweiten Reaktorstufe 16b prozesstechnisch nachgelagert eine zweite Kondensationsstufe 18b zur Abtrennung von in der zweiten Reaktorstufe 16b umgewandeltem Methanol 1 und zum Erhalten des Restgasstroms 9 aus der zweiten Reaktorstufe 16b ist. Auch die zweite Kondensationsstufe 18b ist von der Methanol-Reaktoranordnung 8 umfasst und ebenso kann auch hier das Methanol 1 als ein Rohmethanol abgetrennt werden, welches - wie in der Fig. 1 dargestellt - der obigen Destillationsvorrichtung 18c zugeführt wird. Schließlich kann der Restgasstrom 9 auch in einer Wäschervorrichtung 18d, hier ebenfalls von der Methanol-Reaktoranordnung 8 umfasst, einer Wäsche zum Auswaschen von Methanol 1 unterzogen werden.

Wie untenstehend noch genauer beschrieben wird, kann vorteilhafterweise auf einen Kompressor vor der Methanol-Reaktoranordnung 8 verzichtet werden. Um auch einen Kompressor zwischen der Methanol-Reaktoranordnung 8 und der Ammoniak-Reaktoranordnung 11 zu vermeiden, kann - wie in der Fig. 1 dargestellt - ein Kompressor zwischen den Reaktorstufen 16a, b angeordnet sein. Bevorzugt ist daher, dass das unreagierte Restgas 17 aus der ersten Reaktorstufe 16a einem Boosterkompressor 19 zur Druckerhöhung zugeführt wird, bevor es der zweiten Reaktorstufe 16b zugeführt wird. Weiter ist es bevorzugt, dass der Boosterkompressor 19 einen Ammoniaksynthesedruck bereitstellt, mit welchem der wasserstoffhaltige Strom 10 der Ammoniak-Reaktoranordnung 11 zugeführt wird. Dieser Ammoniaksynthesedruck kann hier beispielhaft 90 bar betragen. Mit anderen Worten beruht der Druck, mit welchem der wasserstoffhaltige Strom 10 der Ammoniak-Reaktoranordnung 11 zugeführt wird, auf dem Boosterkompressor 19, sodass also keine andere Vorrichtung zur Druckerhöhung prozesstechnisch zwischen dem Boosterkompressor 19 und der Ammoniak-Reaktoranordnung 11 angeordnet ist. Damit wird der ansonsten übliche Recyclekompressor in einer Methanolanlage in einen Synthesegaskompressor für den Ammoniakprozess umgewandelt.

Mit dem Ammoniaksynthesedruck ist ein Druck des wasserstoffhaltigen Stroms 10 gemeint, welcher für die insbesondere katalytische Umwandlung in Ammoniak 2 ausreichend ist. Dabei reicht es aus, dass bei diesem Ammoniaksynthesedruck lediglich ein geringer Anteil des wasserstoffhaltigen Stroms 10 in Ammoniak 2 umgewandelt wird. Speziell kann es sein - wie untenstehend noch genauer beschrieben wird - dass nach Zufuhr zu der Ammoniak-Reaktoranordnung 11 bei diesem vergleichsweise niedrigen Druck nach Durchlauf einer ersten Reaktorstufe in der Ammoniak-Reaktoranordnung 11 der verbliebene Stoff aus dem wasserstoffhaltigen Strom 10 innerhalb der Ammoniak-Reaktoranordnung 11 zur Ammoniaksynthese auf einen höheren Druck komprimiert wird. Das Bereitstellen dieses Ammoniaksynthesedrucks durch Druckerhöhung des unreagierten Restgases 17 mittels des Boosterkompressors 19 - und damit eine indirekte Druckerhöhung des erst prozesstechnisch nachgelagert erhaltenen Wasserstoffs - ist deshalb besonders geschickt, weil eine direkte Kompression von im Wesentlichen reinen Wasserstoff praktisch kaum möglich ist. Es ist weiter bevorzugt, dass der Boosterkompressor 19 das unreagierte Restgas 17 auf einen Ammoniaksynthesedruck von mindestens 60 bar erhöht, vorzugsweise auf einen Ammoniaksynthesedruck zwischen 70 bar und 120 bar erhöht. Dabei ist es nicht erforderlich, dass speziell dieses Druckniveau durchgehend von dem Boosterkompressor 19 bis zur Ammoniak-Reaktoranordnung 11 beibehalten wird, vielmehr ist ein gewisser Druckverlust bis dahin möglich.

Wieder speziell das Gewinnen des Synthesegasstroms 7 beschreibend ist es bevorzugt vorgesehen, dass in der Synthesegasreaktoranordnung 6 durch eine autotherme Reformierung mit dem Sauerstoffstrom 4 als Oxidationsmittel der Synthesegasstrom 7 gewonnen wird. Bei der an sich aus dem Stand der Technik bekannten autothermen Reformierung stellt eine katalytische partielle Oxidation - hier mittels des Sauerstoffstroms 4 - die für die endothermen Dampfreformierungsreaktionen erforderliche Wärme bereitstellt. Endotherm ist insbesondere die Reformierung von Methan.

Bekannt ist, dass die autotherme Reformierung zu einer Stöchiometriezahl von weniger als 2 führt, sodass sie an sich zum Erreichen der oben vorgeschlagenen Bereiche für die Stöchiometriezahl nicht geeignet ist. Es hat sich aber überraschend als vorteilhaft erwiesen, eine Wasserstoffanreicherung zur Erhöhung der Stöchiometriezahl vor der Zuführung zur Methanol-Reaktoranordnung 8 vorzusehen, da durch den möglichen Betrieb der autothermen Reformierung bei höheren Drücken ein Synthesegaskompressor vor der Methanolsynthese entfallen kann. Entsprechend ist es bevorzugt, dass die Synthesegasreaktoranordnung 6 einen Methanolsynthesedruck bereitstellt, mit welchem der Synthesegasstrom 7 der Methanol-Reaktoranordnung 8 zugeführt wird. In Analogie zu den Feststellungen zu dem obigen Boosterkompressor 19 bedeutet dies, dass der Druck, mit welchem der Synthesegasstrom 7 der Methanol-Reaktoranordnung 8 zugeführt wird, auf der Synthesegasreaktoranordnung 6 beruht, sodass also keine andere Vorrichtung zur Druckerhöhung prozesstechnisch zwischen der Synthesegasreaktoranordnung 6 und der Methanol-Reaktoranordnung 8 angeordnet ist. Mit dem Methanolsynthesedruck ist ein Druck des Synthesegasstroms 7 gemeint, welcher für die insbesondere katalytische Umwandlung in Methanol 1 ausreichend ist. Speziell kann dieser Methanolsynthesedruck etwa 80 bar betragen.

Hierzu ist es bevorzugt, dass die Synthesegasreaktoranordnung 6 mit einem Druck zwischen 60 bar und 110 bar betrieben wird. Das ist regelmäßig dann nicht zweckmäßig, wenn zur Synthesegaserzeugung auch eine Dampfreformierung eingesetzt wird. Wie in der Fig. 1 dargestellt ist es jedoch bevorzugt, dass der kohlenstoffhaltige Energieträgerstrom 3 vor der Zuführung zu der Synthesegasreaktoranordnung 6 einem Pre-Reformer 20 zum Aufspalten von Kohlenwasserstoffen mit mindestens zwei Kohlenstoffatomen zugeführt wird. Speziell werden diese in Methan, Wasserstoff und Kohlenstoffoxide aufgespaltet. Der Pre-Reformer 20 ist ebenfalls bevorzugt von der vorschlagsgemäßen Anlage umfasst. Ein solches von dem Pre-Reformer 20 durchgeführtes Pre-Reformierung ist an sich aus dem Stand der Technik als Dampfreformierung bei niedriger Temperatur bekannt, wobei sich ebenfalls bekanntermaßen in dem Pre-Reformer 20 die Methanisierungsreaktion und die Wassergas-Shift-Reaktion im Wesentlichen im Gleichgewicht befinden. Als solche Kohlenwasserstoffe mit mindestens zwei Kohlenstoffatomen kommen insbesondere Ethan, Propan, Butan und Pentan infrage. Der Einsatz eines solchen Pre-Reformers, welcher im Gegensatz zu einem Dampfreformer nicht befeuert werden muss, schränkt jedoch - ebenfalls im Gegensatz zu dem Dampfreformer - die Druckverhältnisse nicht wesentlich ein.

Im ersten Ausführungsbeispiel des vorschlagsgemäßen Verfahrens zur kombinierten Herstellung von Methanol 1 und Ammoniak 2 weist der kohlenstoffhaltige Energieträgerstrom 3 vor Zuführung zum Pre-Reformer 20 im Wesentlichen einen jeweiligen molaren Anteil von 63 % an Methan und von 25 % an Wasser auf, wobei der Rest im Wesentlichen aus Ethan und Propan gebildet wird. Der aus dem Pre-Reformer 20 der Synthesegasreaktoranordnung 6 zugeführte kohlenstoffhaltige Energieträgerstrom 3 weist dann - insbesondere durch Zufuhr von Wasserdampf im Pre-Reformer 20 - im ersten Ausführungsbeispiel des vorschlagsgemäßen Verfahrens zur kombinierten Herstellung von Methanol 1 und Ammoniak 2 bei einem Druck von im Wesentlichen 81 bar und einer Temperatur von 645 °C im Wesentlichen einen jeweiligen molaren Anteil von 53 % an Wasser, von 36 % an Methan, von 7 % an Wasserstoff und von 3 % an Kohlenstoffdioxid auf. Der Massenstrom des kohlenstoffhaltigen Energieträgerstroms an dieser Stelle beträgt im ersten Ausführungsbeispiel des vorschlagsgemäßen Verfahrens zur kombinierten Herstellung von Methanol 1 und Ammoniak 2 371.732 kg/h.

Bevorzugt ist vorgesehen, dass der kohlenstoffhaltige Energieträgerstrom 3 Methan aufweist und dass ein Gewinnen von Wasserstoff und Kohlenstoffoxiden im Synthesegasstrom 7 aus dem Methan des Energieträgerstroms 3 überwiegend und vorzugsweise im Wesentlichen vollständig durch die autotherme Reformierung mit dem Sauerstoffstrom 4 als Oxidationsmittel in der Synthesegasreaktoranordnung 6 erfolgt. Überwiegend bedeutet hier speziell, dass mindestens 90 % des Wasserstoff und der Kohlenstoffoxide im Synthesegasstrom 7 aus dem Methan des Energieträgerstroms 3 durch die autotherme Reformierung erfolgt. Der verbliebene Anteil kann beispielsweise auf die an sich geringe Reformierungstätigkeit bei Methan des Pre-Reformers 20 zurückgehen. Bei dieser Variante ist also ein anderer Mechanismus, wie etwa die Dampfreformierung, zur Gewinnung von wesentlichem Wasserstoff und Kohlenstoffoxiden aus dem Methan der Energieträgerstroms 3 ausgeschlossen, sodass effektiv nur eine autotherme Reformierung stattfindet. Im ersten Ausführungsbeispiel des vorschlagsgemäßen Verfahrens zur kombinierten Herstellung von Methanol 1 und Ammoniak 2 weist der Synthesegasstrom 7 aus der Synthesegasreaktoranordnung 6 bei einem Druck von 80 bar und einer Temperatur von 1025 °C im Wesentlichen einen jeweiligen molaren Anteil von 44 % Wasserstoff, von 32 % Wasser, von 16 % Kohlenstoffmonoxid, von 6 % Kohlenstoffdioxid und von 1 % Methan auf.

Wenn auch ein Recyclen des Synthesegases bzw. der Kohlenstoffoxide bei der Methanol-Reaktoranordnung 8 entfallen kann, so stellt ein Recyclen des Wasserstoffes eine bevorzugte mögliche Variante dar. Speziell kann es sein, dass - wie in der Fig. 1 dargestellt - ein erster Wasserstoff-Teilstrom 22a des wasserstoffhaltigen Stromes 10 zur Methanol-Reaktoranordnung 8 und dort insbesondere zur ersten Reaktorstufe 16a rückgeführt wird. Eine Druckerhöhung ist dabei nicht erforderlich, weswegen es bevorzugt ist, dass der erste Wasserstoff-Teilstrom 22a im Wesentlichen mit dem Druck zur ersten Reaktorstufe 16a zugeführt wird, mit welchem der wasserstoffhaltige Strom 10 aus der PSA 21 gewonnen wird. Wie ebenfalls in der Fig. 1 dargestellt wird dann vorzugsweise ein zweiter Wasserstoff-Teilstrom 22b des wasserstoffhaltigen Stroms 10 der Ammoniak-Reaktoranordnung 11 zugeführt.

Es kann sein, dass der Restgasstrom 9 im Wesentlichen vollständig der PSA 21 zum Gewinnen des wasserstoffhaltigen Stroms 10 zugeführt wird. Es kann aber auch - wie in der Fig. 1 dargestellt - sein, dass ein Restgasteilstrom 23 des Restgasstroms 9 einer - insbesondere von der vorschlagsgemäßen Anlage umfassten - Desulfurierungsanordnung 24a zur Entschwefelung des kohlenstoffhaltigen Energieträgerstroms 3 durch Hydrierung zugeführt wird. Prozesstechnisch zwischen der Desulfurierungsanordnung 24a und dem Pre-Reformer 20 kann - wie in der Fig. 1 dargestellt - auch eine Sättigung 24b angeordnet sein.

Die Ausgestaltung der Ammoniak-Reaktoranordnung 11 ist im Grunde beliebig. Prinzipiell ebenso beliebig ist die Quelle des Stickstoffs für die Ammoniaksynthese. Bevorzugt ist zunächst, dass ein aus der Sauerstoffgewinnungsanordnung 5 - hier also aus der Luftzerlegung - gewonnener Stickstoffstrom 25 ebenfalls der Ammoniak-Reaktoranordnung 11 zugeführt wird. Der in dem Synthesegas und bei der Methanolsynthese vorteilhafterweise fehlende Stickstoff kann auf diese Weise wieder in den Prozessstrom eingeführt werden.

Die in der Fig. 1 dargestellte beispielhafte Ammoniak-Reaktoranordnung 11 weist einen erste Ammoniak-Reaktorstufe 26a zur Synthese von Ammoniak 2 auf, welche im "once-through"-Betrieb ("Einmaldurchgang") arbeitet. Die erste Ammoniak-Reaktorstufe 26a arbeitet - da wie oben beschrieben die Kompression von dem Boosterkompressor 19 bereitgestellt wird - in einem Niederdruckbereich. Auf eine erste Abscheidevorrichtung 27a zum Abtrennen von Ammoniak 2 folgt ein Hauptkompressor 28 zur Druckerhöhung auf hier etwa 150 bar, dem eine Loop-Reaktoranordnung - also eine Reaktoranordnung im Kreislaufbetrieb - nachgeschaltet ist.

Speziell ist dem Hauptkompressor 28 eine zweite Ammoniak-Reaktorstufe 26b und eine dritte Ammoniak-Reaktorstufe 26c zur jeweiligen Synthese von Ammoniak 2 prozesstechnisch seriell nachgeschaltet. Zum Schließen des Kreislaufbetriebs wird der Restgasstrom aus einer zweiten Abscheidevorrichtung 27b zum Abtrennen von Ammoniak 2 mittels eines Recyclekompressors 29 wieder der zweiten Ammoniak-Reaktorstufe 26b zugeführt. Aufgrund des höheren Drucks in der zweiten Ammoniak-Reaktorstufe 26b und der dritten Reaktorstufe 26c erfolgt in diesen die Umsetzung zu Ammoniak mit einer höheren Rate als in der ersten Reaktorstufe 26a mit dem niedrigeren Druck.

Die vorschlagsgemäße Anlage dient der kombinierten Herstellung von Methanol 1 und Ammoniak 2, und zwar nach dem vorschlagsgemäßen Verfahren. Die vorschlagsgemäße Anlage umfasst die Sauerstoffgewinnungsanordnung 5 zum Gewinnen des Sauerstoffstroms 4, wobei der Sauerstoffanteil an dem Sauerstoffstrom 4 mindestens 80% beträgt, umfasst die Synthesegasreaktoranordnung 6 zum Gewinnen des Synthesegasstroms 7, welcher Wasserstoff und Kohlenstoffoxide aufweist, aus dem kohlenstoffhaltigen Energieträgerstrom 3 und dem Sauerstoffstrom 4, umfasst die Methanol-Reaktoranordnung 8 zur teilweisen Umwandlung des Synthesegasstroms 7 in Methanol 1 und zum Erhalten des Restgasstroms 9 und umfasst die Ammoniak-Reaktoranordnung 11, welcher der wasserstoffhaltige Strom 10 zumindest teilweise zugeführt wird, welcher wasserstoffhaltige Strom 10 aus dem Restgasstrom 9 gewonnen wurde, und in welcher Ammoniak-Reaktoranordnung 11 der wasserstoffhaltige Strom 10 zumindest teilweise in Ammoniak 2 umgewandelt wird.

Die vorschlagsgemäße Anlage ist dadurch gekennzeichnet, dass vor Zuführung zu der Methanol-Reaktoranordnung 8 der molare Anteil an Wasserstoff des aus der Synthesegasreaktoranordnung 6 gewonnenen Synthesegasstroms 7 gegenüber dem molaren Anteil an Kohlenstoffoxiden in einer Anreicherungsanordnung 30 erhöht wird. Vorzugsweise umfasst die Anreicherungsanordnung die Kohlenstoffdioxidwäsche 12 sowie alternativ oder zusätzlich die Shift-Konvertierung 14 sowie ggf. das Steuerventil 15. Darüber hinaus weist die Anlage eine Druckwechsel-Adsorptionsanlage (PSA) 21 auf, welcher der Restgasstrom 9 zum Gewinnen des wasserstoffhaltigen Stroms 10 zugeführt wird. Diese PSA 21, welche von der vorschlagsgemäßen Anlage umfasst ist, kann auch zum Ausschleusen eines Purgegases dienen.

Merkmale, Eigenschaften und bevorzugte Ausgestaltungen der vorschlagsgemäßen Anlage ergeben sich aus den entsprechenden Merkmalen, Eigenschaften und bevorzugten Ausgestaltungen des vorschlagsgemäßen Verfahrens zur kombinierten Herstellung von Methanol 1 und Ammoniak 2 oder dem vorschlagsgemäßen Verfahren zum Herstellen eines Kraftstoffs und umgekehrt.

Grundsätzlich kann das aus dem vorschlagsgemäßen Verfahren oder aus der vorschlagsgemäßen Anlage hergestellte Methanol 1 einer beliebigen Verwendung zugeführt werden, wobei auch eine Zwischenlagerung sowie ein Transport des Methanols 1 vorgesehen sein kann. Die Herstellung eines Kraftstoffs aus dem auf diese Weise gewonnenen Methanol 1 bietet sich aber besonders an.

Das vorschlagsgemäße Verfahren zum Herstellen eines Kraftstoffs ist folglich dadurch gekennzeichnet, dass der Kraftstoff aus Methanol 1 gewonnen wird, welches Methanol 1 nach dem vorschlagsgemäßen Verfahren zur kombinierten Herstellung von Methanol 1 und von Ammoniak 2 hergestellt wurde.

Eine erste bevorzugte Variante sieht vor, dass der Kraftstoff durch Beimischung des Methanols 1 zu insbesondere herkömmlich hergestelltem Motorenbenzin hergestellt wird. Folglich ist eine Menge Motorenbenzin bereits vorhanden, welchem Motorenbenzin dann das Methanol 1 beigemischt wird. Vorzugsweise weist der so gewonnene Kraftstoff einen Volumenanteil von 10 % bis 20 %, vorzugsweise von im Wesentlichen 15 % an beigemischtem Methanol auf.

Eine zweite bevorzugte Variante ist dadurch gekennzeichnet, dass das Methanol 1 zu einem Derivat verarbeitet wird, und der Kraftstoff durch Beimischung des Derivates zu Motorenbenzin hergestellt wird.

Bei einer weiteren bevorzugten Variante ist vorgesehen, dass das Methanol 1 in den Kraftstoff umgewandelt wird. Hier kann einerseits das Methanol in Dimethylether (DME) umgewandelt werden, welcher Dimethylether dann gemäß einer Möglichkeit als Kraftstoff verwendet wird. Bevorzugt ist es jedoch, dass der Dimethylether nur ein Zwischenprodukt bei der weiteren Umwandlung zu einem aromatenreichen Kohlenwasserstoffgemisch ist. Eine entsprechende bevorzugte Ausführungsform ist dadurch gekennzeichnet, dass das Methanol insbesondere mittels Zeolith-Katalysatoren über das Zwischenprodukt Dimethylether in ein aromatenreiches Kohlenwasserstoffgemisch umgewandelt wird, welches Kohlenwasserstoffgemisch bevorzugt den Kraftstoff bildet.

Eine weitere bevorzugte Variante ist dadurch gekennzeichnet, dass das Methanol direkt mittels Zeolith-Katalysatoren in ein aromatenreiches Kohlenwasserstoffgemisch umgewandelt wird. Hier kann einerseits das Kohlenwasserstoffgemisch den Kraftstoff bilden. Andererseits kann das Kohlenwasserstoffgemisch einem vorhandenen Kraftstoff beigemischt werden, wobei dann die entstehende Mischung den hergestellten Kraftstoff bildet. Weiter ist es möglich, dass das Methanol direkt mittels Zeolith-Katalysatoren in einem gekühlten Wirbelschichtreaktor in das aromatenreiche Kohlenwasserstoffgemisch umgewandelt wird.

Weitere bevorzugte Varianten des vorschlagsgemäßen Verfahrens zum Herstellen eines Kraftstoffs ergeben sich aus den bevorzugten Varianten des vorschlagsgemäßen Verfahrens zur kombinierten Herstellung von Methanol 1 und von Ammoniak 2 sowie aus den bevorzugten Varianten der vorschlagsgemäßen Anlage zur kombinierten Herstellung von Methanol 1 und Ammoniak 2.

Gemäß dem ersten Ausführungsbeispiel des vorschlagsgemäßen Verfahrens zur kombinierten Herstellung von Methanol 1 und Ammoniak 2, welches nachstehend weiter beschrieben wird, wird ein Erdgasstrom mit einem Massenstrom von insgesamt 137.924,5 kg/h bei einer Temperatur von 20 °C und einem Druck von im Wesentlichen 87 bar bereitgestellt.

Der oben bereits beschriebene Synthesegasstrom 7, welcher nach Shiftkonvertierung 13 und Kohlenstoffdioxidwäsche 12 der Methanol-Reaktoranordnung 8 zugeführt wird, weist einen Massenstrom von insgesamt 241.818 kg/h auf. Zusätzlich wird der erste Wasserstoff-Teilstrom 22a aus der PSA 21 der Methanol-Reaktoranordnung 8 zugeführt, wobei dieser im Wesentlichen zu 100 % aus Wasserstoff bei 43 °C und bei im Wesentlichen 87 bar besteht und einen Massenstrom von 26.482 kg/h aufweist.

Unmittelbar bei Austritt aus der Synthesegasreaktoranordnung 6 - und vor der Zufuhr zu einem hier nicht dargestellten Abhitzekessel - weist der Synthesegasstrom 7 - wie oben bereits beschrieben - im Wesentlichen einen jeweiligen molaren Anteil von 44 % Wasserstoff, 32 % Wasser, 16 % Kohlenstoffmonoxid, 6 % Kohlenstoffdioxid und 1 % Methan bei 1025 °C und 80 bar auf. Der Massenstrom insgesamt beträgt hier 523.265 kg/h. Die Mengendifferenz zwischen diesem Massenstrom und dem der Methanol-Reaktoranordnung 8 zugeführten Massenstrom des Synthesegasstroms 7 begründet sich im Wesentlichen einerseits in dem Auswaschvorgang durch die Kohlenstoffdioxidwäsche 12 und andererseits in der Auskondensierung einer erheblichen Menge Wasser aus dem Synthesegasstrom 7.

Aus der Kohlenstoffdioxidwäsche 12 wird ein kohlenstoffdioxidhaltiger Strom gewonnen, welcher einen molaren Anteil von fast 95 % an Kohlenstoffdioxid aufweist und dabei einen Massenstrom von 76.163 kg/h aufweist.

Der Restgasstrom 9 weist vor Zuführung zu der PSA 21 einen Massenstrom von 90.183 kg/h auf, wobei der Restgasteilstrom 23 davon ca. 50 kg/h ausmacht.

Der zweite Wasserstoff-Teilstrom 22b aus der PSA 21 wird gemeinsam mit dem Stickstoffstrom 25 der Ammoniak-Reaktoranordnung 11 und dort speziell der ersten Ammoniak-Reaktorstufe 26a zugeführt. Dabei besteht der Stickstoffstrom 25 zu über 99 % aus Stickstoff und weist einen Massenstrom von 62.623 kg/h auf. Der insgesamt zugeführte Gasstrom weist im Wesentlichen einen jeweiligen molaren Anteil von 75 % Wasserstoff und von 25 % Stickstoff mit lediglich 0,02 % Argon auf. Der Druck beträgt im Wesentlichen 87 bar bei einer Temperatur von 40 °C. Der Massenstrom insgesamt beträgt 63.972 kg/h. Aus der Ammoniak-Reaktoranordnung 11 insgesamt wird dann ein Massenstrom von 60.390 kg/h an Ammoniak gewonnen.

Bei einem zweiten Ausführungsbeispiel des vorschlagsgemäßen Verfahrens zur kombinierten Herstellung von Methanol 1 und von Ammoniak 2 wird - abweichend von dem ersten Ausführungsbeispiel - eine möglichst balancierte Herstellung von Methanol und Ammoniak angestrebt. Bis auf die nachfolgend beschriebenen Unterschiede des zweiten Ausführungsbeispiels zum ersten Ausführungsbeispiel des vorschlagsgemäßen Verfahrens zur kombinierten Herstellung von Methanol 1 und von Ammoniak 2 gelten die für das erste Ausführungsbeispiel getroffenen Feststellungen, insbesondere gelten die selben Angaben zum Erdgasstrom wie im ersten Ausführungsbeispiel.

Der aus dem Pre-Reformer 20 der Synthesegasreaktoranordnung 6 zugeführte kohlenstoffhaltige Energieträgerstrom weist dabei - bis auf einen geringfügig höheren Massenstrom von 371.748 kg/h - im Wesentlichen die für das erste Ausführungsbeispiel beschriebenen Temperatur- und Druckverhältnisse sowie molare Anteile auf. Die Angaben aus dem ersten Ausführungsbeispiel gelten grundsätzlich auch für den Synthesegasstrom 7 aus der Synthesegasreaktoranordnung 6, wobei der Massenstrom insgesamt 523.277 kg/h beträgt.

Zum Zweck der obenstehenden balancierten Herstellung ist das Steuerventil 15 so eingestellt, dass - jeweils bei einem Druck von im Wesentlichen 77 bar und einer Temperatur von 320° C - ein Teilstrom 13 mit einem Massenstrom von 209.311 kg/h zur Shiftkonvertierung 14 abgezweigt wird, während um die Shift-konvertierung 14 ein Massenstrom von 313.966 kg/h durch das Steuerventil 15 herumgeführt wird. Sowohl der Teilstrom 13 als auch der übrige, herumgeführte Synthesegasstrom 7 weisen dabei im Wesentlichen einen jeweiligen molaren Anteil von 44 % Wasserstoff, 32 % Wasser, 16 % Kohlenstoffmonoxid, 6 % Kohlenstoffdioxid, 1 % Methan und weniger als 1 % Stickstoff auf.

Der nach der Kohlenstoffdioxidwäsche 12 der Methanol-Reaktoranordnung 8 zugeführte Synthesegasstrom 7 weist bei einem Massenstrom von insgesamt 185.661 kg/h und im Wesentlichen einen jeweiligen molaren Anteil von 77 % Wasserstoff, 16 % Kohlenstoffmonoxid, 2 % Kohlenstoffdioxid, 2 % Methan, 2 % Wasser und 1 % Stickstoff auf. Gegenüber dem ersten Ausführungsbeispiel ist also der Anteil an Wasserstoff gegenüber dem Anteil an Kohlenstoffmonoxid erhöht. In diesem Ausführungsbeispiel wird kein erster Wasserstoff-Teilstrom 22a rückgeführt.

Der kohlenstoffdioxidhaltige Strom aus der Kohlenstoffdioxidwäsche 12 weist bei einem molaren Anteil von ebenfalls fast 95 % an Kohlenstoffdioxid einen Massenstrom von 171.906 kg/h auf

Dies führt zu einem Restgasstrom 9 - wiederum vor Zuführung zu der Druckwechsel-Adsorptionsanlage 21 - mit einem jeweiligen molaren Anteil von im Wesentlichen 87 % Wasserstoff, 4 % Kohlenstoffmonoxid, 2 % Kohlenstoffdioxid, 4 % Methan, 1 % Wasser und 2 % Stickstoff und mit einem Massenstrom von 66.893 kg/h, wobei wiederum der Restgasteilstrom 23 ca. 50 kg/h ausmacht.

Der Stickstoffstrom 25 weist hier einen Massenstrom von 89.430 kg/h auf. Der insgesamt der Ammoniak-Reaktoranordnung 11 zugeführte Gasstrom unterscheidet sich gegenüber dem ersten Ausführungsbeispiel lediglich in seinem Massenstrom von insgesamt 108.717 kg/h, wobei dann aus der Ammoniak-Reaktoranordnung 11 insgesamt ein Massenstrom von 105.067 kg/h an Ammoniak gewonnen wird.

Aus der ersten Kondensationsstufe 18a wird ein Massenstrom von ca. 60.318 kg/h Methanol 1 gewonnen. Aus der zweiten Kondensationsstufe 18b und der Wäschervorrichtung 18d wird ein Massenstrom von insgesamt ca. 46.211 kg/h Methanol 1 gewonnen, was mit dem Methanol 1 aus der ersten Kondensationsstufe 18a einen Massenstrom von ca. 106.529 kg/h an Methanol 1 ergibt und damit nur etwa 2/3 des entsprechenden Wertes des ersten Ausführungsbeispiels entspricht. Aus der Destillationsvorrichtung 18c wird dann ein im Wesentlichen aus Methanol bestehender Reinmethanolstrom mit einem Massenstrom von 106.332 kg/h gewonnen. Erkennbar wird in dem zweiten Ausführungsbeispiel mehr Ammoniak und dafür weniger Methanol hergestellt.

Bei einem dritten Ausführungsbeispiel des vorschlagsgemäßen Verfahrens zur kombinierten Herstellung von Methanol 1 und von Ammoniak 2, welches nachstehend beschrieben wird, wird wieder eine gegenüber der Herstellung von Ammoniak bevorzugte Herstellung von Methanol angestrebt. Mit anderen Worten liegt ein Übergewichtung der Herstellung von Methanol gegenüber der Herstellung von Ammoniak vor.

Wiederum gelten - soweit nicht anders vermerkt - grundsätzlich die Ausführungen zu dem ersten Ausführungsbeispiel des vorschlagsgemäßen Verfahrens.

Der Erdgasstrom ist identisch. Der aus dem Pre-Reformer 20 der Synthesegasreaktoranordnung 6 zugeführte kohlenstoffhaltige Energieträgerstrom 3 weist einen Massenstrom von 371.734 kg/h auf. Der Synthesegasstrom 7 aus der Synthesegasreaktoranordnung 6 weist einen Massenstrom von insgesamt 523.266 kg/h auf.

Es findet eine Abzweigung durch das Steuerventil 15 statt. Speziell wird ein Teilstrom 13 mit einem Massenstrom von 52.326 kg/h zur Shiftkonvertierung 14 abgezweigt und ein Massenstrom von 470.939 kg/h durch das Steuerventil 15 um die Shiftkonvertierung 14 herumgeführt, wobei die Druck- und Temperaturverhältnisse sowie die jeweiligen molaren Anteile wie bei dem zweiten Ausführungsbeispiel des Verfahrens gelten.

Der nach der Kohlenstoffdioxidwäsche 12 der Methanol-Reaktoranordnung 8 zugeführte Synthesegasstrom 7 weist einen Massenstrom von insgesamt 227.778 kg/h und im Wesentlichen einen jeweiligen molaren Anteil von 70 % Wasserstoff, 22 % Kohlenstoffmonoxid, 2 % Kohlenstoffdioxid, 2 % Methan, 2 % Wasser und 1 % Stickstoff auf.

Der kohlenstoffdioxidhaltige Strom aus der Kohlenstoffdioxidwäsche 12 weist bei einem molaren Anteil von wiederum fast 95 % an Kohlenstoffdioxid einen Massenstrom von 100.098 kg/h auf.

Die Folge ist ein Restgasstrom 9 vor Zuführung zu der Druckwechsel-Adsorptionsanlage 21 mit einem jeweiligen molaren Anteil von im Wesentlichen 90 % Wasserstoff, 4 % Kohlenstoffmonoxid, 1 % Kohlenstoffdioxid, 2 % Methan, 1 % Wasser und 1 % Stickstoff. Der Massenstrom beträgt 85.447 kg/h, wobei ca. 50 kg/h auf den Restgasteilstrom 23 entfallen.

Der Stickstoffstrom 25 weist einen Massenstrom von 41.450 kg/h auf. Der insgesamt der Ammoniak-Reaktoranordnung 11 zugeführte Gasstrom weist einen Massenstrom von insgesamt 50.390 kg/h auf, wobei dann aus der Ammoniak-Reaktoranordnung 11 insgesamt ein Massenstrom von 46.835 kg/h an Ammoniak gewonnen wird.

Aus der ersten Kondensationsstufe 18a wird ein Massenstrom von ca. 86.300 kg/ Methanol1 gewonnen. Aus der zweiten Kondensationsstufe 18b und der Wäschervorrichtung 18d wird ein Massenstrom von insgesamt ca. 65.586 kg/h Methanol 1 gewonnen, was mit dem Methanol 1 aus der ersten Kondensationsstufe 18a einen Massenstrom von ca. 151.886 kg/h an Methanol 1 ergibt. Aus der Destillationsvorrichtung 18c wird dann ein im Wesentlichen aus Methanol bestehender Reinmethanolstrom mit einem Massenstrom von 151.670 kg/h gewonnen.

## Patentansprüche

1. Verfahren zur kombinierten Herstellung von Methanol (1) und von Ammoniak (2), wobei ein kohlenstoffhaltiger Energieträgerstrom (3) und ein Sauerstoffstrom (4) aus einer Sauerstoffgewinnungsanordnung (5) einer Synthesegasreaktoranordnung (6) zum Gewinnen eines Synthesegasstroms (7) mit Wasserstoff und Kohlenstoffoxiden zugeführt wird, wobei der Synthesegasstrom (7) einer Methanol-Reaktoranordnung (8) zur teilweisen Umwandlung in Methanol (1) zugeführt wird, wobei aus der Methanol-Reaktoranordnung (8) ein Restgasstrom (9) erhalten wird, aus welchem ein wasserstoffhaltiger Strom (10) gewonnen wird, welcher zumindest teilweise einer Ammoniak-Reaktoranordnung (11) zugeführt und dort zumindest teilweise in Ammoniak (2) umgewandelt wird und wobei der Sauerstoffanteil an dem Sauerstoffstrom (4) mindestens 80 % beträgt, **dadurch gekennzeichnet, dass** wobei in einem Anreicherungsschritt der molare Anteil an Wasserstoff des aus der Synthesegasreaktoranordnung (6) gewonnenen Synthesegasstroms (7) gegenüber dem molaren Anteil an Kohlenstoffoxiden vor Zuführung zu der Methanol-Reaktoranordnung (8) erhöht wird und dass der Restgasstrom (9) zumindest teilweise einer Druckwechsel-Adsorptionsanlage (PSA) (21) zum Gewinnen des wasserstoffhaltigen Stroms (10) zugeführt wird.

2. Verfahren nach Anspruch 1, **dadurch gekennzeichnet, dass** in dem Anreicherungsschritt Kohlenstoffdioxid zumindest teilweise aus dem Synthesegasstrom (7) entfernt wird, vorzugsweise, dass zumindest ein Teilstrom des Synthesegasstroms (7) einer Kohlenstoffdioxidwäsche (12) zur zumindest teilweisen Entfernung des Kohlenstoffdioxids zugeführt wird, insbesondere, dass der der Methanol-Reaktoranordnung (8) zugeführte Synthesegasstrom (7) einen molaren Anteil von Kohlenstoffdioxid von bis zu 3 % aufweist.

3. Verfahren nach Anspruch 1 oder 2, **dadurch gekennzeichnet, dass** in dem Anreicherungsschritt zumindest ein, insbesondere weiterer, Teilstrom (13) des Synthesegasstroms (7) einer Shiftkonvertierung (14) zugeführt wird, in welcher Kohlenstoffmonoxid zumindest teilweise durch Reaktion mit Wasserdampf zu Wasserstoff und Kohlenstoffdioxid umgewandelt wird, vorzugsweise, dass die Abzweigung des Teilstroms (13) zur Shiftkonvertierung (14) der Kohlenstoffdioxidwäsche (12) prozesstechnisch vorgelagert ist, weiter vorzugsweise, dass der der Shiftkonvertierung (14) zugeführte Teilstrom (13) anschließend der Kohlenstoffdioxidwäsche (12) zugeführt wird.

4. Verfahren nach Anspruch 3, **dadurch gekennzeichnet, dass** die Shift-konvertierung (14) im Wesentlichen ausschließlich eine Hochtemperatur-Wassergas-Shift-Reaktion, vorzugsweise bei mindestens 450°, durchführt, weiter vorzugsweise, dass der molare Anteil des der Shiftkonvertierung (14) zugeführten insbesondere weiteren Teilstroms (13) am Synthesegasstrom (7) zur Einstellung des Verhältnisses des durch Umwandlung gewonnenen Methanols (1) und des durch Umwandlung gewonnen Ammoniaks (2) angepasst wird.

5. Verfahren nach einem der Ansprüche 1 bis 4, **dadurch gekennzeichnet, dass** Kohlenstoffmonoxid im der Methanol-Reaktoranordnung (8) zugeführten Synthesegasstrom (7) im Wesentlichen vollständig in Methanol (1) umgewandelt wird, sodass ein molarer Anteil von Kohlenstoffmonoxid im Restgasstrom (9) höchstens 4 % ist.

6. Verfahren nach einem der Ansprüche 1 bis 5, **dadurch gekennzeichnet, dass** die Methanol-Reaktoranordnung (8) eine erste Reaktorstufe (16a) zur Synthese von Methanol (1) und eine zweite Reaktorstufe (16b) zur Synthese von Methanol (1) aufweist, dass der der Methanol-Reaktoranordnung (8) zugeführte Synthesegasstrom (7) der ersten Reaktorstufe (16a) zugeführt wird und dass ein unreagiertes Restgas (17) aus der ersten Reaktorstufe (16a) der zweiten Reaktorstufe (16b) zugeführt wird, vorzugsweise, dass der ersten Reaktorstufe (16a) ein erstes Fluid zu seiner Kühlung zugeführt wird und/oder dass der zweiten Reaktorstufe (16b) ein zweites Fluid zu seiner Kühlung zugeführt wird, insbesondere, dass die erste Reaktorstufe (16a) isotherm betrieben wird und/oder dass die zweite Reaktorstufe (16b) isotherm betrieben wird.

7. Verfahren nach Anspruch 6, **dadurch gekennzeichnet, dass** das der zweiten Reaktorstufe (16b) zugeführte unreagierte Restgas (17) im Wesentlichen alle unreagierten Kohlenstoffoxide aus der ersten Reaktorstufe (16a) umfasst, vorzugsweise, dass Kohlenstoffoxide des Synthesegasstroms (7) die erste Reaktorstufe (16a) nur einmal durchlaufen,

8. Verfahren nach Anspruch 6 oder 7, **dadurch gekennzeichnet, dass** der Restgasstrom (9) im Wesentlichen alle unreagierten Kohlenstoffoxide aus der zweiten Reaktorstufe (16b) umfasst, vorzugsweise, dass Kohlenstoffoxide die zweite Reaktorstufe (16b) nur einmal durchlaufen.

9. Verfahren nach einem der Ansprüche 6 bis 8, **dadurch gekennzeichnet, dass** der ersten Reaktorstufe (16a) prozesstechnisch nachgelagert eine erste Kondensationsstufe (18a) zur Abtrennung von in der ersten Reaktorstufe (16a) umgewandeltem Methanol (1) und zum Erhalten des unreagierten Restgases (17) aus der ersten Reaktorstufe (16a) ist, vorzugsweise, dass der zweiten Reaktorstufe (16b) prozesstechnisch nachgelagert eine zweite Kondensationsstufe (18b) zur Abtrennung von in der zweiten Reaktorstufe (16b) umgewandeltem Methanol (1) und zum Erhalten des Restgasstroms (9) aus der zweiten Reaktorstufe (16b) ist

10. Verfahren nach einem der Ansprüche 6 bis 9, **dadurch gekennzeichnet, dass** das unreagierte Restgas (17) aus der ersten Reaktorstufe (16a) einem Boosterkompressor (19) zur Druckerhöhung zugeführt wird bevor es des zweiten Reaktorstufe (16b) zugeführt wird, vorzugsweise, dass der Boostkompressor (19) einen Ammoniaksynthesedruck bereitstellt, mit welchem der wasserstoffhaltige Strom (10) der Ammoniak-Reaktoranordnung (11) zugeführt wird, insbesondere, dass der Boosterkompressor (19) das unreagierte Restgas (17) auf einen Ammoniaksynthesedruck von mindestens 60 bar erhöht, vorzugsweise auf einen Ammoniaksynthesedruck zwischen 70 bar und 120 bar erhöht.

11. Verfahren nach einem der Ansprüche 1 bis 10, **dadurch gekennzeichnet, dass** in der Synthesegasreaktoranordnung (6) durch eine autotherme Reformierung mit dem Sauerstoffstrom (4) als Oxidationsmittel der Synthesegasstrom (7) gewonnen wird, vorzugsweise, dass die Synthesegasreaktoranordnung (6) einen Methanolsynthesedruck bereitstellt, mit welchem der Synthesegasstrom (7) der Methanol-Reaktoranordnung (8) zugeführt wird, insbesondere, dass die Synthesegasreaktoranordnung (6) mit einem Druck zwischen 60 bar und 110 bar betrieben wird, weiter vorzugsweise, dass der kohlenstoffhaltige Energieträgerstrom (3) vor der Zuführung zu der Synthesegasreaktoranordnung (6) einem Pre-Reformer (20) zum Aufspalten von Kohlenwasserstoffen mit mindestens zwei Kohlenstoffatomen zugeführt wird.

12. Verfahren nach Anspruch 11, **dadurch gekennzeichnet, dass** der kohlenstoffhaltige Energieträgerstrom (3) Methan aufweist und dass ein Gewinnen von Wasserstoff und Kohlenstoffoxiden im Synthesegasstrom (7) aus dem Methan des Energieträgerstroms (3) überwiegend, vorzugsweise im Wesentlichen vollständig, durch die autotherme Reformierung mit dem Sauerstoffstrom (4) als Oxidationsmittel in der Synthesegasreaktoranordnung (6) erfolgt.

13. Verfahren nach einem der Ansprüche 1 bis 12, **dadurch gekennzeichnet, dass** ein erster Wasserstoff-Teilstrom (22a) des wasserstoffhaltigen Stromes (10) zur Methanol-Reaktoranordnung (8), insbesondere zur ersten Reaktorstufe (16a), rückgeführt wird, weiter vorzugsweise, dass der erste Wasserstoff-Teitstrom im Wesentlichen mit dem Druck zur ersten Reaktorstufe zugeführt wird, mit welchem der wasserstoffhaltige Strom (10) aus der PSA gewonnen wird, weiter insbesondere, dass ein zweiter Wasserstoff-Teilstrom (22b) des wasserstoffhaltigen Stroms (10) der Ammoniak-Reaktoranordnung (11) zugeführt wird.

14. Verfahren nach einem der Ansprüche 1 bis 13, **dadurch gekennzeichnet, dass** ein Restgasteilstrom (23) des Restgasstroms (9) einer Desulfurierungsanordnung (24a) zur Entschwefelung des kohlenstoffhaltigen Energieträgerstroms (3) durch Hydrierung zugeführt wird.

15. Verfahren nach einem der Ansprüche 1 bis 14, **dadurch gekennzeichnet, dass** in einem weiteren Verfahrensschritt das Methanol (1) zu einem Kraftstoff umgesetzt wird, vorzugsweise, dass das Methanol (1) insbesondere mittels Zeolith-Katalysatoren in ein aromatenreiches Kohlenwasserstoffgemisch umgewandelt wird, weiter vorzugsweise, welches Kohlenwasserstoffgemisch den Kraftstoff bildet oder als Kraftstoffzusatz einem vorhandenen Kraftstoff beigemischt wird.

16. Anlage zur kombinierten Herstellung von Methanol (1) und Ammoniak (2), insbesondere nach einem Verfahren gemäß den Ansprüchen 1 bis 14, mit einer Sauerstoffgewinnungsanordnung (5) zum Gewinnen eines Sauerstoffstroms (4), wobei der Sauerstoffanteil an dem Sauerstoffstrom (4) mindestens 80 % beträgt, mit einer Synthesegasreaktoranordnung (6) zum Gewinnen eines Synthesegasstroms (7) mit Wasserstoff und Kohlenstoffoxiden aus einem kohlenstoffhaltigen Energieträgerstrom (3) und dem Sauerstoffstrom (4), mit einer Methanol-Reaktoranordnung (8) zur teilweisen Umwandlung des Synthesegasstroms (7) in Methanol (1) und zum Erhalten eines Restgasstroms (9) und mit einer Ammoniak-Reaktoranordnung (11), welcher ein wasserstoffhaltiger Strom (10) zumindest teilweise zugeführt wird, welcher aus dem Restgasstrom (9) gewonnen wurde, und in welcher der wasserstoffhaltige Strom (10) zumindest teilweise in Ammoniak (2) umgewandelt wird, **dadurch gekennzeichnet, dass** vor Zuführung zu der Methanol-Reaktoranordnung (8) der molare Anteil an Wasserstoff des aus der Synthesegasreaktoranordnung (6) gewonnenen Synthesegasstroms (7) gegenüber dem molaren Anteil an Kohlenstoffoxiden in einer Anreicherungsanordnung (30) erhöht wird und dass die Anlage eine Druckwechsel-Adsorptionsanlage (PSA) 21 aufweist, welcher der Restgasstrom (9) zum Gewinnen des wasserstoffhaltigen Stroms (10) zumindest teilweise zugeführt wird.

## Claims

1. A method for the combined production of methanol (1) and of ammonia (2), wherein a carbon-containing energy carrier flow (3) and an oxygen flow (4) from an oxygen-producing assembly (5) are fed to a synthesis gas reactor assembly (6) for obtaining a synthesis gas flow (7) with hydrogen and carbon oxides, wherein the synthesis gas flow (7) is fed to a methanol reactor assembly (8) for the partial conversion into methanol (1), wherein, from the methanol reactor assembly (8), a residual gas flow (9) is obtained from which a hydrogen-containing flow (10) is obtained, which is at least partially fed to an ammonia reactor assembly (11) and at least partially converted into ammonia (2) there, and wherein the oxygen fraction of the oxygen flow (4) is at least 80%, **characterized in that**, in an enrichment step, the molar fraction of hydrogen in the synthesis gas flow (7) obtained from the synthesis gas reactor assembly (6) is increased in relation to the molar fraction of carbon oxides prior to the feeding to the methanol reactor assembly (8), and that the residual gas flow (9) is at least partially fed to a pressure swing adsorption system (PSA) (21) for obtaining the hydrogen-containing flow (10).

2. The method according to claim 1, **characterized in that**, in the enrichment step, carbon dioxide is at least partially removed from the synthesis gas flow (7), preferably, that at least a partial flow of the synthesis gas flow (7) is fed to carbon dioxide scrubbing (12) for at least partially removing the carbon dioxide, in particular, that the synthesis gas flow (7) fed to the methanol reactor assembly (8) has a molar fraction of carbon dioxide of up to 3%.

3. The method according to claim 1 or 2, **characterized in that**, in the enrichment step, at least one, in particular further, partial flow (13) of the synthesis gas flow (7) is fed to a shift conversion (14), in which carbon monoxide is at least partially converted into hydrogen and carbon dioxide by a reaction with water vapor, preferably, that the branching-off of the partial flow (13) to the shift conversion (14), with respect to the process, is arranged upstream of the carbon dioxide scrubbing (12), more preferably, that the partial flow (13) fed to the shift conversion (14) is subsequently fed to the carbon dioxide scrubbing (12).

4. The method according to claim 3, **characterized in that** the shift conversion (14) substantially exclusively carries out a high-temperature water-gas shift reaction, preferably at at least 450°, further preferably, that the molar fraction in the synthesis gas flow (7) of the, in particular further, partial flow (13) fed to the shift conversion (14) is adapted for adjusting the ratio of the methanol (1) obtained by conversion and the ammonia (2) obtained by conversion.

5. The method according to any one of the claims 1 to 4, **characterized in that** carbon monoxide in the synthesis gas flow (7) fed to the methanol reactor assembly (8) is substantially completely converted into methanol (1), so that a molar fraction of carbon monoxide in the residual gas flow (9) is 4% at most.

6. The method according to any one of the claims 1 to 5, **characterized in that** the methanol reactor assembly (8) has a first reactor stage (16a) for the synthesis of methanol (1) and a second reactor stage (16b) for the synthesis of methanol (1), that the synthesis gas flow (7) fed to the methanol reactor assembly (8) is fed to the first reactor stage (16a), and that an unreacted residual gas (17) from the first reactor stage (16a) is fed to the second reactor stage (16b), preferably, that a first fluid is fed to the first reactor stage (16a) for cooling it and/or that a second fluid is fed to the second reactor stage (16b) for cooling it, in particular, that the first reactor stage (16a) is operated isothermally and/or that the second reactor stage (16b) is operated isothermally.

7. The method according to claim 6, **characterized in that** the unreacted residual gas (17) fed to the second reactor stage (16b) substantially includes all unreacted carbon oxides from the first reactor stage (16a), preferably, that carbon oxides of the synthesis gas flow (7) pass through the first reactor stage (16a) only once.

8. The method according to claim 6 or 7, **characterized in that** the residual gas flow (9) substantially includes all unreacted carbon oxides from the second reactor stage (16b), preferably, that carbon oxides pass through the second reactor stage (16b) only once.

9. The method according to any one of the claims 6 to 8, **characterized in that**, with respect to the process, a first condensation stage (18a) for the separation of methanol (1) converted in the first reactor stage (16a) and for obtaining the unreacted residual gas (17) from the first reactor stage (16a) is arranged downstream of the first reactor stage (16a), preferably, that, with respect to the process, a second condensation stage (18b) for the separation of methanol (1) converted in the second reactor stage (16b) and for obtaining the residual gas flow (9) from the second reactor stage (16b) is arranged downstream of the second reactor stage (16b).

10. The method according to any one of the claims 6 to 9, **characterized in that** the unreacted residual gas (17) from the first reactor stage (16a) is fed to a booster compressor (19) for increasing pressure prior to feeding it to the second reactor stage (16b), preferably, that the booster compressor (19) provides an ammonia synthesis pressure with which the hydrogen-containing flow (10) is fed to the ammonia reactor assembly (11), in particular, that the booster compressor (19) increases the unreacted residual gas (17) to an ammonia synthesis pressure of at least 60 bars, preferably to an ammonia synthesis pressure between 70 bars and 120 bars.

11. The method according to any one of the claims 1 to 10, **characterized in that** the synthesis gas flow (7) is obtained in the synthesis gas reactor assembly (6) by autothermic reforming with the oxygen flow (4) as an oxidant, preferably, that the synthesis gas reactor assembly (6) provides a methanol synthesis pressure with which the synthesis gas flow (7) is fed to the methanol reactor assembly (8), in particular, that the synthesis gas reactor assembly (6) is operated with a pressure of between 60 bars and 110 bars, further preferably, that the carbon-containing energy carrier flow (3), prior to being fed to the synthesis gas reactor assembly (6), is fed to a pre-reformer (20) for splitting hydrocarbons with at least two carbon atoms.

12. The method according to claim 11, **characterized in that** the carbon-containing energy carrier flow (3) includes methane, and that hydrogen and carbon oxides in the synthesis gas flow (7) from the methane of the energy carrier flow (3) are obtained predominantly, preferably substantially completely, by autothermic reforming with the oxygen flow (4) as the oxidant in the synthesis gas reactor assembly (6).

13. The method according to any one of the claims 1 to 12, **characterized in that** a first hydrogen partial flow (22a) of the hydrogen-containing flow (10) is returned to the methanol reactor assembly (8), in particular to the first reactor stage (16a), further preferably, that the first hydrogen partial flow is substantially fed to the first reactor stage with the pressure with which the hydrogen-containing flow (10) is obtained from the PSA, further in particular, that a second hydrogen partial flow (22b) of the hydrogen-containing flow (10) is fed to the ammonia reactor assembly (11).

14. The method according to any one of the claims 1 to 13, **characterized in that** a residual gas partial flow (23) of the residual gas flow (9) is fed to a desulfurization assembly (24a) for desulfurizing the carbon-containing energy carrier flow (3) by hydrogenation.

15. The method according to any one of the claims 1 to 14, **characterized in that** the methanol (1) is converted into a fuel in a further method step, preferably, that the methanol (1) is converted into an aromatics-rich hydrocarbon mixture, in particular by means of zeolite catalysts, further preferably, which hydrocarbon mixture forms the fuel or is admixed as a fuel additive to an existing fuel.

16. A system for the combined production of methanol (1) and of ammonia (2), in particular in accordance with a method according to the claims 1 to 14, comprising an oxygen-producing assembly (5) for obtaining an oxygen flow (4), wherein the oxygen fraction of the oxygen flow (4) is at least 80%, a synthesis gas reactor assembly (6) for obtaining a synthesis gas flow (7) with hydrogen and carbon oxides from a carbon-containing energy carrier flow (3) and the oxygen flow (4), a methanol reactor assembly (8) for the partial conversion of the synthesis gas flow (7) into methanol (1) and for obtaining a residual gas flow (9), and an ammonia reactor assembly (11), to which a hydrogen-containing flow (10) that was obtained from the residual gas flow (9) is at least partially fed, and in which the hydrogen-containing flow (10) is at least partially converted into ammonia (2), **characterized in that**, prior to the feeding to the methanol reactor assembly (8), the molar fraction of hydrogen in the synthesis gas flow (7) obtained from the synthesis gas reactor assembly (6) is increased in relation to the molar fraction of carbon oxides in an enrichment assembly (30), and that the system comprises a pressure swing adsorption system (PSA) (21) to which the residual gas flow (9) is at least partially fed in order to obtain the hydrogen-containing flow (10).

## Revendications

1. Procédé de production combinée de méthanol (1) et d'ammoniac (2), dans lequel un courant porteur d'énergie carboné (3) et un courant d'oxygène (4) provenant d'un ensemble de récupération d'oxygène (5) est amené à un ensemble de réacteur de gaz de synthèse (6) pour obtenir un courant de gaz de synthèse (7) avec de l'hydrogène et des oxydes de carbone, dans lequel le courant de gaz de synthèse (7) est amené à un ensemble de réacteur de méthanol (8) pour une conversion partielle en méthanol (1), dans lequel un courant de gaz résiduel (9) est obtenu à partir de l'ensemble de réacteur de méthanol (8), à partir duquel est obtenu un courant contenant de l'hydrogène (10) qui est amené au moins en partie à un ensemble de réacteur d'ammoniac (11) et y est au moins en partie converti en ammoniac (2), et dans lequel la teneur en oxygène du courant d'oxygène (4) est d'au moins 80 %, **caractérisé par le fait que**, dans une étape d'enrichissement, la proportion molaire d'hydrogène du courant de gaz de synthèse (7) obtenu à partir de l'ensemble de réacteur de gaz de synthèse (6) est augmentée par rapport à la proportion molaire d'oxydes de carbone avant qu'il soit amené à l'ensemble de réacteur de méthanol (8) et que le courant de gaz résiduel (9) est au moins en partie amené à une installation d'adsorption à pression modulée (PSA) (21) pour récupérer le courant contenant de l'hydrogène (10).

2. Procédé selon la revendication 1, **caractérisé par le fait que** dans l'étape d'enrichissement le dioxyde de carbone est au moins en partie éliminé du courant de gaz de synthèse (7), de préférence qu'au moins un courant partiel du courant de gaz de synthèse (7) est amené à un lavage de dioxyde de carbone (12) pour une élimination au moins partielle du dioxyde de carbone, en particulier que le courant de gaz de synthèse (7) amené à l'ensemble de réacteur de méthanol (8) présente une proportion molaire de dioxyde de carbone allant jusqu'à 3 %.

3. Procédé selon la revendication 1 ou 2, **caractérisé par le fait que** dans l'étape d'enrichissement au moins un, en particulier un autre courant partiel (13) du courant de gaz de synthèse (7) est amené à une conversion catalytique (14) dans laquelle le monoxyde de carbone est converti au moins en partie en hydrogène et en dioxyde de carbone par réaction avec de la vapeur d'eau, de préférence que la dérivation du courant partiel (13) pour la conversion catalytique (14) est située en amont du lavage de dioxyde de carbone (12) quant au processus, en outre de préférence que le courant partiel (13) amené à la conversion catalytique (14) est amené ensuite au lavage de dioxyde de carbone (12).

4. Procédé selon la revendication 3, **caractérisé par le fait que** la conversion catalytique (14) effectue pour l'essentiel exclusivement une réaction du gaz à l'eau à haute température, de préférence à au moins 450°, en outre de préférence que la proportion molaire dudit en particulier autre courant partiel (13) amené à la conversion catalytique (14), au courant de gaz de synthèse (7), est adapté pour ajuster le rapport du méthanol (1) obtenu par conversion et de l'ammoniac (2) obtenu par conversion.

5. Procédé selon l'une quelconque des revendications 1 à 4, **caractérisé par le fait que** le monoxyde de carbone dans le courant de gaz de synthèse (7) amené à l'ensemble de réacteur de méthanol (8) est converti pour l'essentiel complètement en méthanol (1) de sorte qu'une proportion molaire de monoxyde de carbone dans le courant de gaz résiduel (9) est de 4 % tout au plus.

6. Procédé selon l'une quelconque des revendications 1 à 5, **caractérisé par le fait que** l'ensemble de réacteur de méthanol (8) comprend un premier étage de réacteur (16a) pour la synthèse du méthanol (1) et un deuxième étage de réacteur (16b) pour la synthèse du méthanol (1), que le courant de gaz de synthèse (7) amené à l'ensemble de réacteur de méthanol (8) est amené au premier étage de réacteur (16a) et qu'un gaz résiduel n'ayant pas réagi (17) provenant du premier étage de réacteur (16a) est amené au deuxième étage de réacteur (16b), de préférence que le premier étage de réacteur (16a) est alimenté en un premier fluide pour son refroidissement et/ou que le deuxième étage de réacteur (16b) est alimenté en un deuxième fluide pour son refroidissement, en particulier que le premier étage de réacteur (16a) fonctionne de manière isotherme et/ou que le deuxième étage de réacteur (16b) fonctionne de manière isotherme.

7. Procédé selon la revendication 6, **caractérisé par le fait que** le gaz résiduel (17) n'ayant pas réagi qui est amené au deuxième étage de réacteur (16b) comprend pour l'essentiel tous les oxydes de carbone n'ayant pas réagi provenant du premier étage de réacteur (16a), de préférence que des oxydes de carbone du courant de gaz de synthèse (7) ne traversent qu'une seule fois le premier étage de réacteur (16a).

8. Procédé selon la revendication 6 ou 7, **caractérisé par le fait que** le courant de gaz résiduel (9) comprend pour l'essentiel tous les oxydes de carbone n'ayant pas réagi provenant du deuxième étage de réacteur (16b), de préférence que des oxydes de carbone ne traversent qu'une seule fois le deuxième étage de réacteur (16b).

9. Procédé selon l'une quelconque des revendications 6 à 8, **caractérisé par le fait qu'**un premier étage de condensation (18a) destiné à séparer le méthanol (1) converti dans le premier étage de réacteur (16a) et à obtenir le gaz résiduel (17) n'ayant pas réagi provenant du premier étage de réacteur (16a) est situé, quant au processus, en aval du premier étage de réacteur (16a), de préférence qu'un deuxième étage de condensation (18b) destiné à séparer le méthanol (1) converti dans le deuxième étage de réacteur (16a) et à obtenir le courant de gaz résiduel (9) provenant du deuxième étage de réacteur (16a) est situé, quant au processus, en aval du deuxième étage de réacteur (16b).

10. Procédé selon l'une quelconque des revendications 6 à 9, **caractérisé par le fait que** le gaz résiduel (17) n'ayant pas réagi provenant du premier étage de réacteur (16a) est amené à un compresseur booster (19) pour augmenter la pression avant qu'il soit amené au deuxième étage de réacteur (16b), de préférence que le compresseur booster (19) fournit une pression de synthèse d'ammoniac à laquelle le courant contenant de l'hydrogène (10) est amené à l'ensemble de réacteur d'ammoniac (11), en particulier que le compresseur booster (19) fait passer le gaz résiduel (17) n'ayant pas réagi à une pression de synthèse d'ammoniac d'au moins 60 bars, de préférence à une pression de synthèse d'ammoniac comprise entre 70 bars et 120 bars.

11. Procédé selon l'une quelconque des revendications 1 à 10, **caractérisé par le fait que** le courant de gaz de synthèse (7) est obtenu dans l'ensemble de réacteur de gaz de synthèse (6) par un reformage autothermique avec le courant d'oxygène (4) en tant qu'agent oxydant, de préférence que ledit ensemble de réacteur de gaz de synthèse (6) fournit une pression de synthèse de méthanol à laquelle le courant de gaz de synthèse (7) est amené à l'ensemble de réacteur de méthanol (8), en particulier que l'ensemble de réacteur de gaz de synthèse (6) fonctionne à une pression comprise entre 60 bars et 110 bars, en outre, de préférence, que le courant porteur d'énergie contenant du carbone (3) est amené à un pré-reformeur (20) pour séparer les hydrocarbures avec au moins deux atomes de carbone, avant qu'il soit amené à l'ensemble de réacteur de gaz de synthèse (6).

12. Procédé selon la revendication 11, **caractérisé par le fait que** le courant porteur d'énergie carboné (3) comprend du méthane et qu'une récupération d'hydrogène et d'oxydes de carbone dans le courant de gaz de synthèse (7) à partir du méthane du courant porteur d'énergie (3) se fait majoritairement, de préférence pour l'essentiel complètement, par le reformage autothermique avec le courant d'oxygène (4) comme agent oxydant dans l'ensemble de réacteur de gaz de synthèse (6).

13. Procédé selon l'une quelconque des revendications 1 à 12, **caractérisé par le fait qu'**un premier courant partiel d'hydrogène (22a) du courant contenant de l'hydrogène (10) est ramené à l'ensemble de réacteur de méthanol (8), en particulier au premier étage de réacteur (16a), en outre de préférence que le premier courant partiel d'hydrogène est amené au premier étage de réacteur pour l'essentiel à la pression à laquelle le courant contenant de l'hydrogène (10) est obtenu à partir de la PSA, en outre en particulier qu'un deuxième courant partiel d'hydrogène (22b) du courant contenant de l'hydrogène (10) est amené à l'ensemble de réacteur d'ammoniac (11).

14. Procédé selon l'une quelconque des revendications 1 à 13, **caractérisé par le fait qu'**un courant partiel de gaz résiduel (23) du courant de gaz résiduel (9) est amené à un ensemble de désulfuration (24a) pour désulfurer par hydrogénation le courant porteur d'énergie carboné (3).

15. Procédé selon l'une quelconque des revendications 1 à 14, **caractérisé par le fait que**, dans une autre étape du procédé, le méthanol (1) est transformé en un carburant, de préférence que le méthanol (1) est transformé en un mélange d'hydrocarbures riche en aromatiques, en particulier au moyen de catalyseurs zéolithiques, en outre de préférence, lequel mélange d'hydrocarbures forme le carburant ou est mélangé comme additif de carburant à un carburant existant.

16. Installation de production combinée de méthanol (1) et d'ammoniac (2), en particulier selon un procédé selon les revendications 1 à 14, comprenant un ensemble de récupération d'oxygène (5) pour récupérer un courant d'oxygène (4), la teneur en oxygène dans le courant d'oxygène (4) étant d'au moins 80 %, un ensemble de réacteur de gaz de synthèse (6) pour obtenir un courant de gaz de synthèse (7) avec de l'hydrogène et des oxydes de carbone à partir d'un courant porteur d'énergie carboné (3) et du courant d'oxygène (4), un ensemble de réacteur de méthanol (8) pour une conversion partielle du courant de gaz de synthèse (7) en méthanol (1) et pour obtenir un courant de gaz résiduel (9), ainsi qu'un ensemble de réacteur d'ammoniac (11) auquel est amené au moins en partie un courant contenant de l'hydrogène (10) qui a été obtenu à partir du courant de gaz résiduel (9), et dans lequel le courant contenant de l'hydrogène (10) est au moins partiellement converti en ammoniac (2), **caractérisé par le fait que**, avant l'amenée à l'ensemble de réacteur de méthanol (8), la proportion molaire d'hydrogène du courant de gaz de synthèse (7) obtenu à partir de l'ensemble de réacteur de gaz de synthèse (6) est augmentée par rapport à la proportion molaire d'oxydes de carbone dans un ensemble d'enrichissement (30) et que l'installation comprend une installation d'adsorption à pression modulée (PSA) (21) à laquelle est amené au moins en partie le courant de gaz résiduel (9) pour obtenir le courant contenant de l'hydrogène (10).
